# EUROPEAN PATENT SPECIFICATION

(11) **EP 4 345 453 B1**
(45) Date of publication and mention of the grant of the patent: **12.11.2025**
(21) Application number: 23214072.3
(22) Date of filing: 26.09.2019
(51) Int. Cl.: G01N 33/50, C07K 14/74

(54) **HIGH THROUGHPUT EPITOPE IDENTIFICATION AND T CELL RECEPTOR SPECIFICITY DETERMINATION USING LOADABLE DETECTION MOLECULES**
EPITOPIDENTIFIZIERUNG MIT HOHEM DURCHSATZ UND T-ZELL-REZEPTOR-SPEZIFITÄTSBESTIMMUNG MITHILFE BELADBARER DETEKTIONSMOLEKÜLE
IDENTIFICATION D'ÉPITOPE À RENDEMENT ÉLEVÉ ET DÉTERMINATION DE LA SPÉCIFICITÉ DU RÉCEPTEUR DES LYMPHOCYTES T AU MOYEN DE MOLÉCULES DE DÉTECTION CHARGEABLES

(30) Priority: 28.09.2018 EP 18197682
(43) Date of publication of application: 03.04.2024
(62) Divisional of application: 19773108.6
(73) Proprietor: 10X Genomics, Inc., Pleasanton, CA 94588-3260 (US)
(72) Inventor: HADRUP, Sine Reker, 2830 Virum (DK); JAKOBSEN, Søren Nybroe, 2920 Charlottenlund (DK); SAINI, Sunil Kumar, 2880 Bagsværd (DK)
(74) Representative: J A Kemp LLP

(56) References cited:
- WO-A1-2010/060439
- WO-A1-2017/013170
- WO-A2-2015/188839
- US-A- 6 153 408
- US-B2- 9 494 588

## Description

### Technical field of the invention

The present invention relates to detection of interactions between antigenic peptides and binding partners, such as T cell receptors (TCR) and antigenic peptide responsive T cells. In particular, the present invention relates to the provision of loadable detection molecules with peptide-free MHC molecules utilized for high throughput epitope identification and TCR specificity determination.

### Background of the invention

Major histocompatibility complex class I (MHC-I) proteins display the cellular proteome, as small peptide fragments, at the cell surface to mediate T-cell immune surveillance, which is essential for countering cellular abnormalities, such as viral infections and cancer. The identification and characterization of antigen-specific T cells are of substantial importance for therapeutic development and mechanistic insights into immune-affected diseases. Screening of T cell specificity using large libraries of pMHC molecules is suitable for analyses of T cell recognition potentially at genome-wide levels rather than analyses restricted to a selection of model antigens. Such strategies provide novel insights into the immune specificities involved in disease development and response to immunotherapy, and extend fundamental knowledge related to T cell recognition patterns and cross-recognition by TCRs.

Previous protocols has sought to expand the capacity of MHC multimer-based detection technologies to facilitate large-scale epitope discovery and immune monitoring in limited biological material.

Hadrup et al. (2009) and WO 2010/060439 A1 described an approach founded on combinatorial encoding of fluorescently labelled MHC multimers. In this method, each distinct pMHC multimer is assigned with a unique dual color code, which is then used to identify the T cells binding to this particular pMHC molecule. When employing the unique dual color code, any T cell not matching the pre-determined color combinations is recorded as a background event. This combicoding approach yields a low detection limit and enables an increased throughput compared to assays based on conventional fluorescent labelled MHC multimers, as the combination of eight fluorophores yields 28 unique dual-color codes.

Bentzen et al. (2016) and WO 2015/188839 A2 describes another approach in which DNA barcodes attached to MHC multimers form specific tags for each pMHC epitope. The DNA barcodes may be designed with complexity of up to 10¹⁰ different unique sequences and detection of antigen-responsive T cells are therefore not limited by the number of available labels using this technique.

Thus, MHC multimer-based T-cell detection approaches have evolved from the detection of few antigen-specific T-cell populations per sample to the identification of more than 1000 specificities in parallel. However, a major constraint that limits the flexibility, rapidity, and quality assurance of these state-of-the-art T-cell detection platforms is the generation of large libraries of different peptide-MHC complexes (pMHC), which are often 100-1000 different pMHC. To overcome this challenge, peptide exchange technologies have been developed to facilitate the generation of multiple pMHC from a single stock of conditional ligand MHC-I for a given HLA or H-2 molecule. These strategies include the use of dipeptides as catalysts for peptide exchange, the widely used UV-mediated peptide exchange technology, and a more recently developed temperature-induced exchange technology. Unfortunately, apart from requiring an additional peptide exchange process step, exchange technologies have disadvantages, including HLA-specific design and optimization, inefficient peptide exchange of low-affinity incoming peptides, the ability to multimerize only after peptide exchange, and extended exchange duration.

Yet another limitation of current multimer-based T cell detection approaches is the inherent instability of the major histocompatibility complex which is known to have an antigenic peptide dependent lifetime. The instability of pMHC limits the window in which pMHC remains fully functional and thus useful for T cell detection. Also the instability of the pMHC complex limits the time MHC-multimers can remain fully functional and capable of efficiently and antigen-specifically identifying T cells.

US 9,494,588 describes the provision of a mutated MHC class I molecule that can be produced without antigenic peptide. This empty MHC class I molecule could potentially be used to generate the large libraries of different peptide-MHC complexes (pMHC) required for high throughput MHC-based T-cell detection. However, whether this methodology is applicable for use in such a setting is purely speculative and is not disclosed. *E.g.* their ability to interact with T cells with similar antigen-specific interaction properties as wt MHC molecules has not been demonstrated. Furthermore, it is unclear whether the introduced mutation may structurally or functionally affect the TCR-pMHC interactions, possibly leading to unreliable results that does not reflect the native interaction between antigenic peptide and TCRs or antigenic peptide responsive T cells.

Moreover, all existing technologies for MHC-based T cell detection assays are inconsistent when it comes to detection of rare and weak interactions between antigenic peptide and TCRs or antigenic peptide responsive T cells. As an example, the fluorescence-based readout of the combicoding technique is detected by flow cytometry and the signal intensity associated with a given pMHC is therefore the only measure distinguishing those T cells that interact specifically with MHC multimers and those that do not. Thus, it is essential that a given T cell binds numerous MHC multimers, to obtain sufficient fluorescence intensity and thereby enable unambiguous identification of their specificity. Consequently, it becomes increasingly more difficult to accurately resolve and discover any antigenic peptide interactions as the interaction becomes weaker and occur less frequent. This may lead to inconsistent results. Therefore, the ability to detect rare and weak interactions, which is especially important for the discovery of cancer-associated antigens and mutation-derived neoepitopes, is presently insufficient. Even more so, the recognition of self-antigens, are often characterized by low-avidity interactions and small T cell populations. To detect such rare populations, which may still contribute to disease development, improved detection techniques are required.

Hence, an improved method for high throughput epitope identification and TCR specificity determination would be advantageous. In particular, a more flexible, sensitive and faster method in which the end-user on-demand can easily generate large antigenic peptide libraries for screening and reliably discover even rare and weak interactions, e.g. of cancer-associated antigens and mutation-derived neoepitopes, would be advantageous.

### Summary of the invention

Thus, an object of the present invention relates to the provision of improved detection molecules that can be utilized for high throughput epitope identification and TCR specificity determination.

In particular, it is an object of the present invention to provide a method that solves the above mentioned problems of the prior art with generation of large libraries of antigenic peptide-MHC complexes (pMHC) and insufficient sensitivity to detect rare and weak interactions.

Thus, one aspect of the invention relates to a method for detection of one or more antigenic peptide responsive T cells in a sample, the method comprising the following steps:
i. providing loadable detection molecules comprising at least one peptide-free MHC class I molecule and at least one detectable label,
ii. providing at least one antigenic peptide,
iii. contacting the loadable detection molecules with the at least one antigenic peptide to provide loaded detection molecules comprising at least one peptide-MHC (pMHC) class I molecule,
iv. contacting the loaded detection molecules with the sample, and
v. detecting binding of the loaded detection molecules to the one or more antigenic peptide responsive T cells,
wherein each of the at least one antigenic peptide is represented by:
- at least two different detectable labels, or
- at least one detectable label which is a nucleic acid label.

Another aspect of the present invention relates to a loadable detection molecule comprising at least one peptide-free MHC class I molecule and a nucleic acid label.

Yet another aspect of the present invention is to provide a composition for detection of one or more antigenic peptide responsive T cells in a sample, the composition comprising:
(a) at least two loadable detection molecules comprising at least one peptide-free MHC class I molecule and a nucleic acid label, or
(b) at least three loadable detection molecules comprising at least one peptide-free MHC class I molecule and at least one detectable label, wherein each loadable detection molecule comprises a different detectable label.

Still another aspect of the present invention relates to the use of a loadable detection molecule comprising at least one peptide-free MHC class I molecule and a nucleic acid label or a composition as described herein for detection of one or more antigenic peptide responsive T cells in a sample.

A further aspect of the present invention is to provide a kit for detection of one or more antigenic peptide responsive T cells in a sample, the kit comprising:
i. a loadable detection molecule comprising at least one peptide-free MHC class I molecule and a nucleic acid label or a composition as described herein,
ii. at least one antigenic peptide, and
iii. optionally, instructions for use.

An even further aspect of the present invention relates to a method for determining the interaction between a T cell receptor (TCR) or antigenic peptide responsive T cell and a library of antigenic peptides, the method comprising the following steps:
i. providing loadable detection molecules comprising at least one peptide-free MHC class I molecule and a nucleic acid label,
ii. providing a library of antigenic peptides,
iii. contacting the loadable detection molecules with the library of antigenic peptides to provide a library of loaded detection molecules comprising peptide-MHC (pMHC) class I molecules,
iv. contacting the T cell receptor (TCR) or antigenic peptide responsive T cell with the library of loaded detection molecules, and
v. detecting binding of the T cell receptor (TCR) or antigenic peptide responsive T cell with the library of loaded detection molecules.

A still further aspect of the present invention relates to the use of a loadable detection molecule comprising at least one peptide-free MHC class I molecule and a nucleic acid label for determining the interaction between a T cell receptor (TCR) or antigenic peptide responsive T cell and a library of antigenic peptides.

### Brief description of the figures

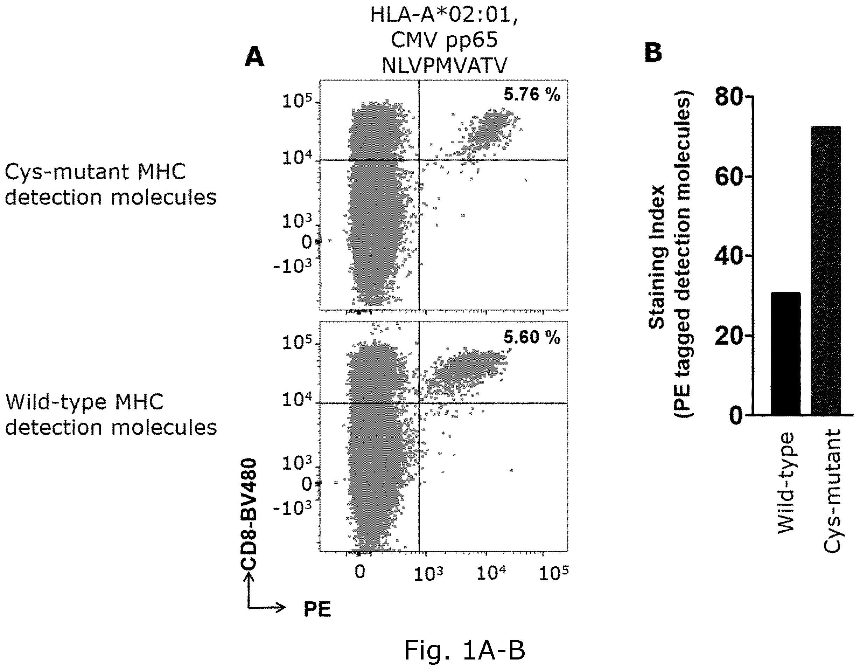

**Figure 1****:** (A) Comparative dot plots of flow cytometry analysis for HLA-A*02:01 (A2) restricted-NLVPMVATV-specific T-cell detection using Cys-mutant MHC and wild-type MHC detection molecules (PE tagged) from healthy donor PBMCs. 2x10⁶ PBMCs were stained with Cys-mutant A2 detection molecules or with wild-type A2 detection molecules specific to A2-NLVPMVATV virus derived antigen. Comparable antigen specific T-cell frequencies (numbers on the dot plots in percentage of total CD8+ cells) were observed but with brighter and better separation using Cys-mutant MHC detection molecules. (B) Staining index comparing Cys-mutant MHC and wild-type MHC detection molecules for NLVPMVATV-specific T-cell detection from the plots in Figure 1A. The staining index was calculated as (Median of Positive - Median of Negative) / (SD of Negative * 2). A threefold higher staining index was detected with Cys-mutant MHC detection molecules.

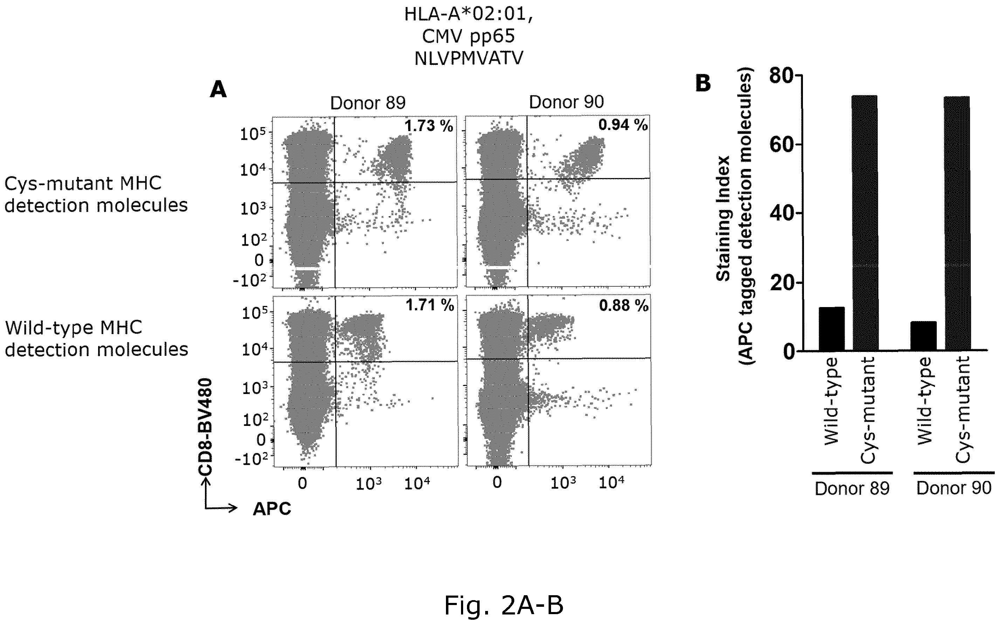

**Figure 2****:** (A) Comparative dot plots of flow cytometry analysis for A2-NLVPMVATV-specific T-cell detection using Cys-mutant MHC and wild-type MHC detection molecules (APC tagged) from two different healthy donor PBMCs. 2x10⁶ PBMCs were stained with Cys-mutant A2 detection molecules or with wild-type A2 detection molecules specific to A2-NLVPMVATV virus derived antigen. Comparable antigen specific T-cell frequencies (numbers on the dot plots in percentage of total CD8+ cells) were observed but again with brighter and better separation using Cys-mutant MHC detection molecules tagged with APC fluorophore. (B) Staining index comparing Cys-mutant MHC and wild-type MHC detection molecules for NLVPMVATV-specific T-cell detection from the plots in Figure 2A. The staining index was calculated as (Median of Positive - Median of Negative) / (SD of Negative * 2). Six to eight fold higher staining index was detected with Cys-mutant MHC detection molecules tagged with APC compared to wild-type MHC detection molecules across the two donor samples.

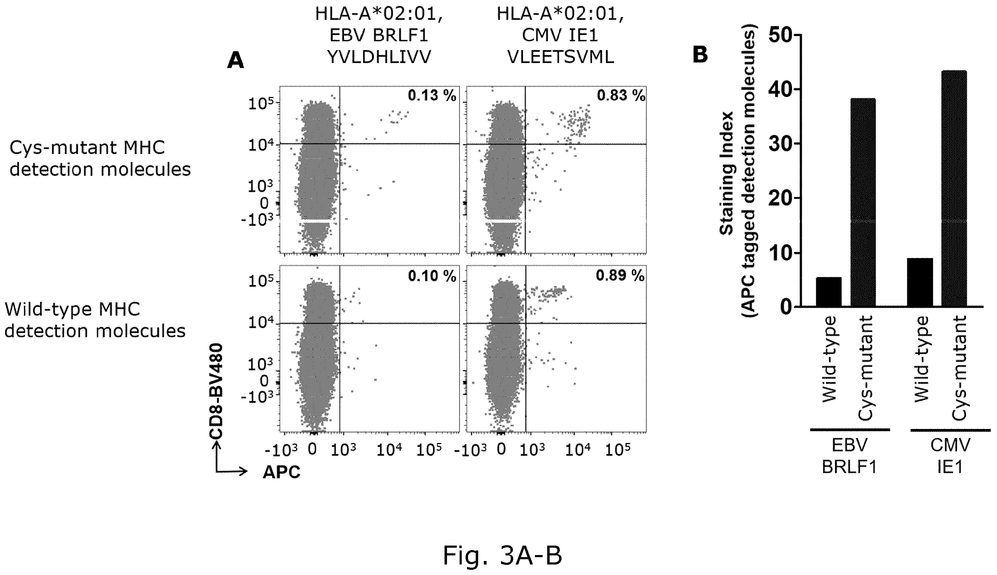

**Figure 3****:** (A) Comparative dot plots of flow cytometry analysis for HLA-A*02:01 (A2) restricted-YVLDHLIVV (EBV BRLF1) and VLEETSVML (CMV IE1) specific T-cell detection using Cys-mutant MHC and wild-type MHC detection molecules (APC tagged) from healthy donor PBMCs. Cys-mutant MHC detection molecules detected both low frequency (A2-YVLDHLIVV) and high frequency (A2-VLEETSVML) CD8+ T cells comparable (numbers on the dot plots in percentage of total CD8+ cells) with wild-type MHC detection molecules but with brighter and better separation. (B) Staining index comparing Cys-mutant MHC and wild-type MHC detection molecules for A2-YVLDHLIVV and A2-VLEETSVML specific T-cell detection from the plots in Figure 3A. Much higher staining index was detected with Cys-mutant MHC detection molecules tagged with APC compared to wild-type MHC detection molecules across both type of antigen specific T cells.

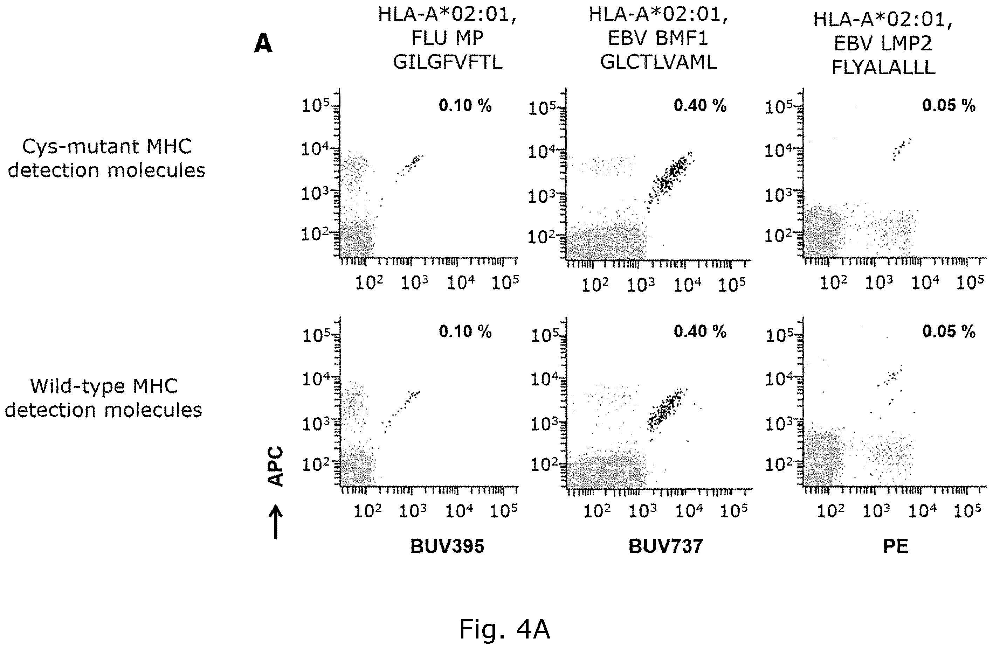

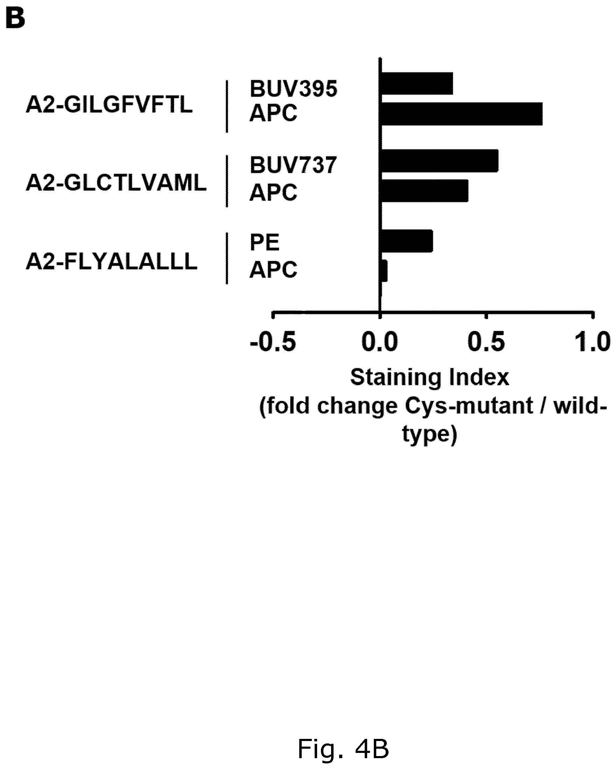

**Figure 4****:** (A) Flow cytometry plot of antigen specific T cells detected using combicoding. Dot plots represent detected antigen specific CD8+ T cells, grey dots represent pMHC detection molecule-negative or single color-positive cells, and black dots represent dual-color detection molecule-positive cells. Dot plots compare T-cells detected using Cys-mutant MHC and wild-type MHC detection molecules in parallel. 2x10⁶ PBMCs healthy donor PBMCs were stained with a pool of two color combicoded detection molecules of different antigen specificities. Identified two color detection molecule-positive CD8 cells are shown in the plots with respective color combinations plotted on X and Y axis. Comparative frequencies (numbers on the dot plots in percentage of total CD8+ cells) of antigen specific T-cell detection were observed between Cys-mutant MHC and wild-type MHC detection molecules. (B) Fold change of staining index, comparing Cys-mutant MHC and wild-type MHC detection molecules for each color label used for the given antigen specific T-cell population detected in Figure 4A. Across all three antigen specific T-cells a higher fold change in staining index was observed for Cys-mutant MHC over wild type MHC detection molecules.

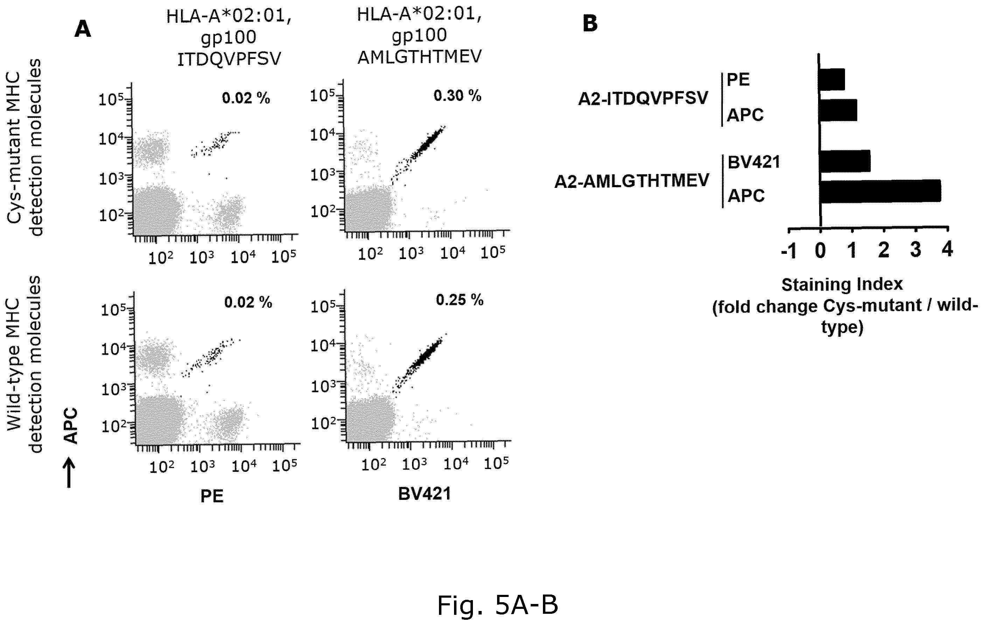

**Figure 5****:** (A) Dot plots representing melanoma associated antigen specific T cells detected using Cys-mutant MHC and wild-type MHC detection molecules from expanded TILs of a melanoma patient. A library of 12 melanoma-associated antigenic peptides was screened using the combicoding method. Dot plots showing two identified T cell populations, with respective color combination, in the melanoma patient sample and comparing identified frequencies (numbers on the dot plots in percentage of total CD8+ cells) between two types of T cell detection molecules. (B) Fold change in staining index (as described for Figure 4B) for melanoma associated antigen specific T cells, shown in the dot plots of Figure 5A. Numbers on dot plots represent the percentage of pMHC multimer-positive cells out of total CD8+ T cells. For both type of antigen specific T-cells a higher fold change in staining index was observed for Cys-mutant MHC over wild type MHC detection molecules.

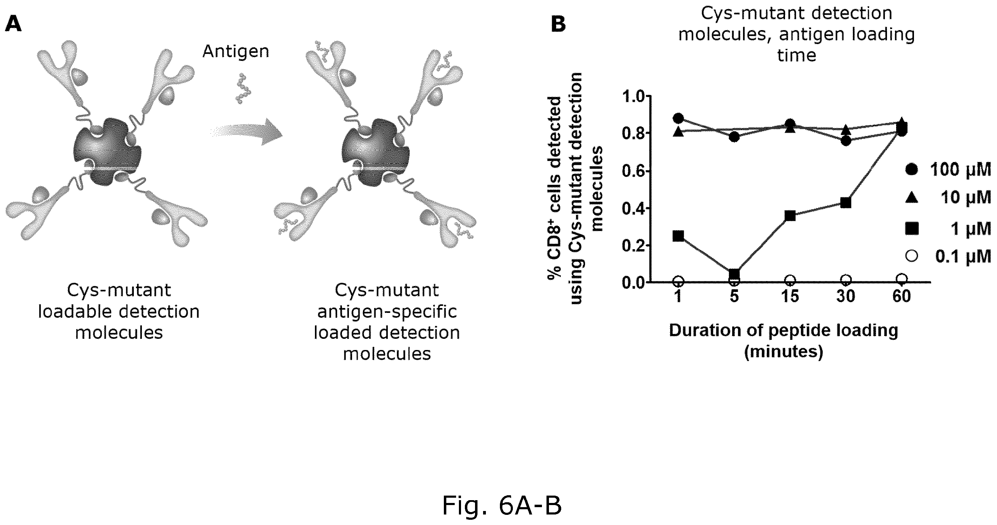

**Figure 6****:** (A) Schematic illustration of loadable detection molecules assembled using Cys-mutant MHC I molecules. Loadable Cys mutant detection molecules were stored at -20°C and loaded with peptide on demand. (B) Peptide loading time and peptide concentration optimization to loadable multimers. FLU MP GILGFVFTL antigen peptide was loaded to Cys-mutant A2 detection molecules for different time points at four different antigen concentrations (0.1, 1, 10, and 100 µM). After each time point with given peptide concentration GILGFVFTL-specific T cells were detected from healthy donor PBMCs by staining 2x10⁶ cells. The frequency, detected as detection molecule-positive CD8+ T cells is plotted against incubation time for each tested peptide concentration. Functional antigen-specific loaded detection molecules can be converted from loadable Cys-mutant MHC detection molecules in just one minute.

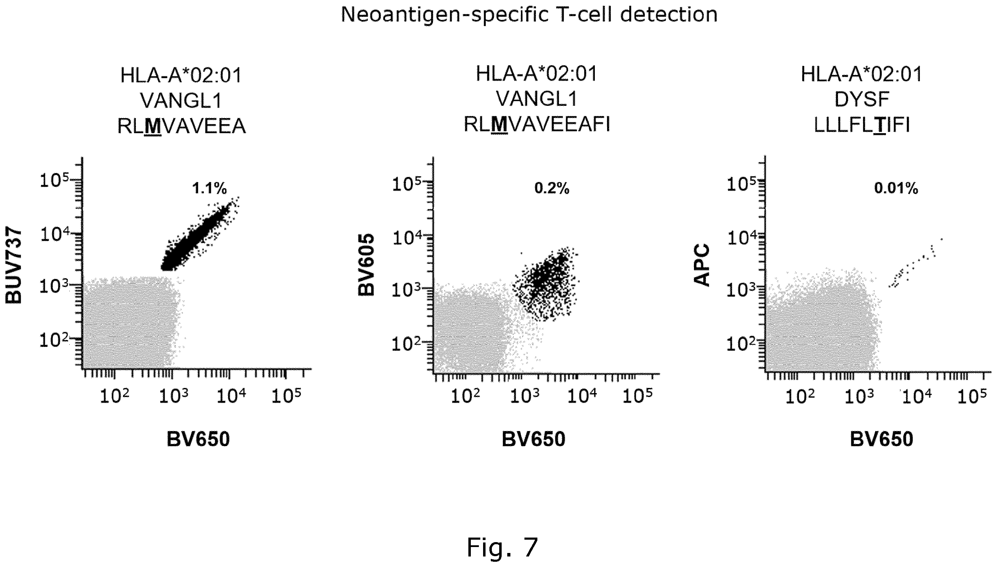

**Figure 7****:** Neoantigen-specific T cells in melanoma TILs identified by the combicoding strategy using loadable Cys-mutant MHC detection molecules. pMHC Cys-mutant detection molecules for 43 A2-restricted peptides (neoantigens) were generated, each in a two-color combination. Loadable Cys-mutant A2 detection molecules were incubated with 100 µM peptide for 15 minutes, followed by staining 2x10⁶ TILs of melanoma patient. Dot plots show three neoantigen-specific T-cell populations identified in this patient sample; each is shown with indicated fluorochromes and peptide sequences. The mutated amino acid position of the neoantigens is underlined and bolded. Numbers on dot plots represent the percentage of pMHC multimer-positive cells out of total CD8+ T cells.

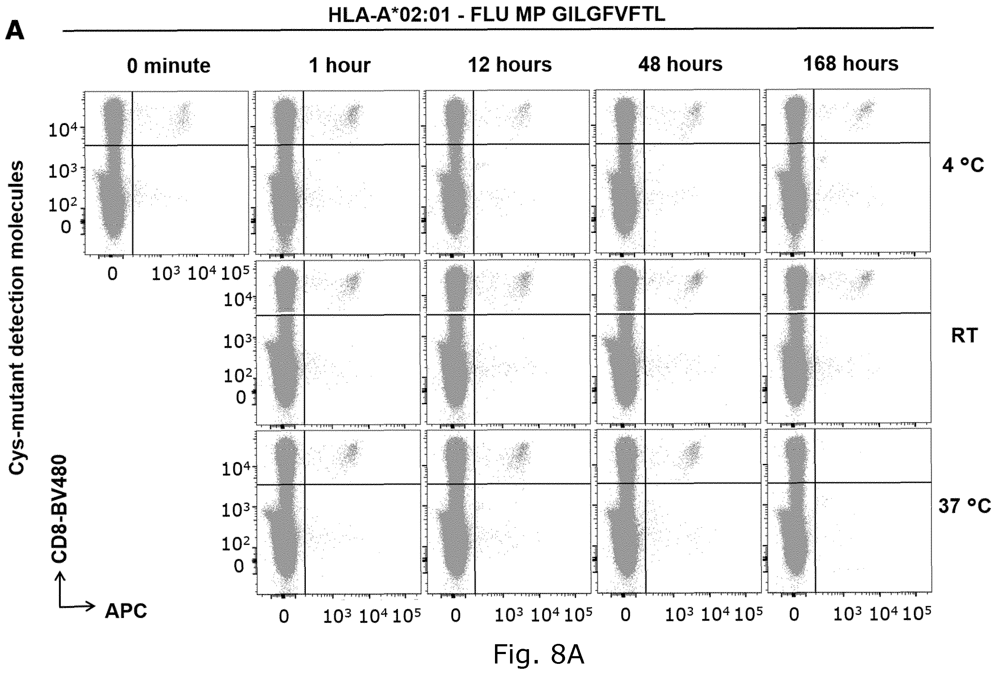

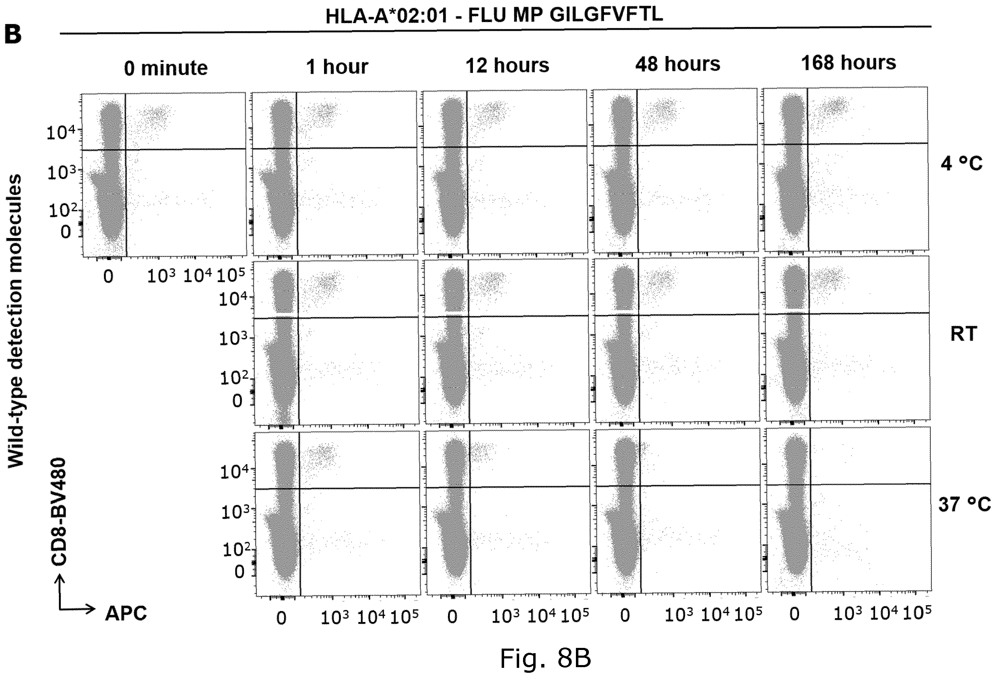

**Figure 8****:** Antigenic peptide-MHC stability (pMHC) compared between detection molecules prepared with Cys-mutant A2 molecules (Figure 8A) or wild type MHC A2 molecules (Figure 8B). A2-GILGGFVFTL (FLU MP)-specific detection molecules were prepared using wild type A2 or Cys-mutant A2 using loadable multimers. Excess peptides were removed after pMHC multimer preparation, using molecular weight cut-off spin columns, and multimers were incubated at three different temperatures for indicated time points, after which T-cell detection efficiency was measured from healthy donor PBMCs. Peptide dissociation rate, measured as T-cell detection efficiency, demonstrate the superior stability of Cys-mutant MHC molecules. At 37°C detection molecules prepared with wild-type MHC A2 shows decreasing T-cell detection efficiency from 12 hour onwards, whereas Cys-mutant A2 detection molecules perform with same detection efficiency at 37°C up to 48 hour time point.

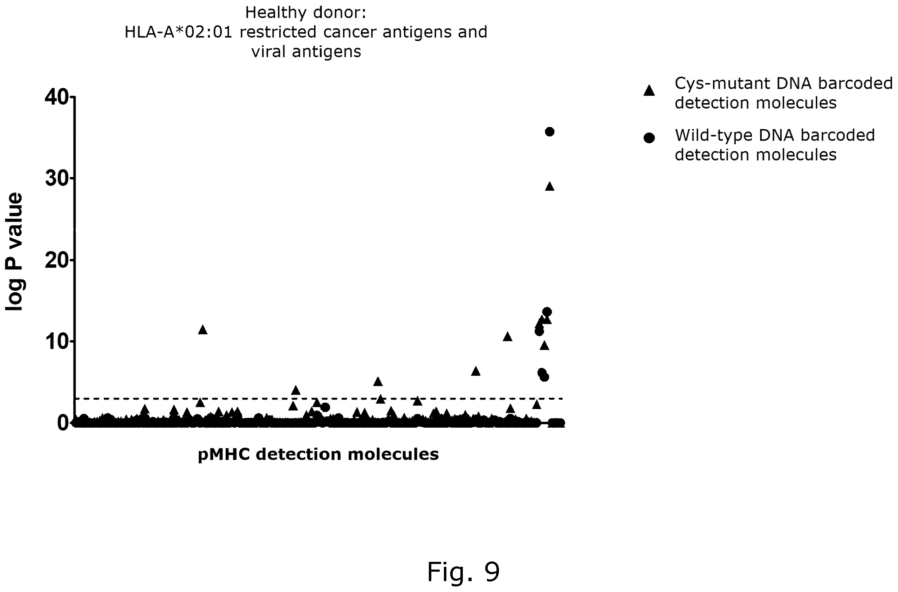

**Figure 9****:** Cys-mutant A2 molecules utilized in high throughput barcoding method for antigen specific T-cell detection. A library of 175 antigens (167 cancer associated antigens, and eight virus derived antigens) was screened to identify antigen specific T-cells from healthy donor PBMCs. Detection molecules were assembled using Cys-mutant A2 or wild type A2 molecules. Each antigen-specific detection molecule was tagged with a unique DNA barcode. Pooled library of 175 antigen specific detection molecules was stained with 5x10⁶ healthy donors PBMCs. Comparative results of detection molecules made from Cys-mutant A2 molecules and wild type A2 molecules for all 175 pMHC specificities (x-axis) are shown on the plot. Data plotted on the y-axis are the -log10(P) (in respect to the pMHC-associated DNA barcode) for the DNA-barcoded detection molecules. Dotted line at x = 3 (-log10(0.001)) represent the selected threshold, all readout above this are considered significant for identified antigen specific T-cells.

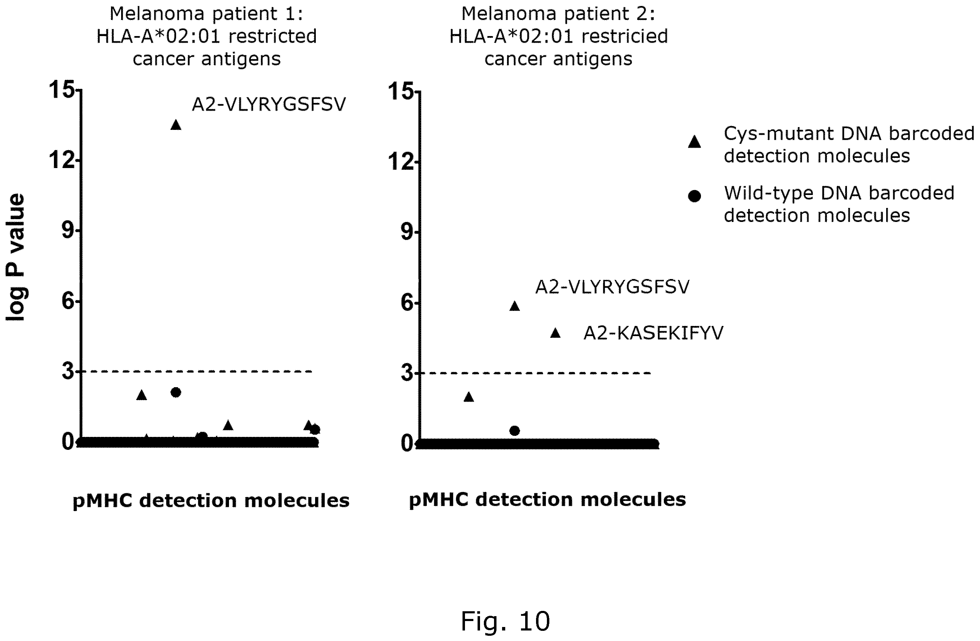

**Figure 10****:** Cancer-associated antigen specific CD8 T-cells identified in high throughput barcoding method using Cys-mutant A2 molecules. A library of 175 antigens (167 cancer associated antigens, and eight virus derived antigens) was screened to identify antigen specific T-cells in two melanoma patient TILs. Detection molecules were assembled using Cys-mutant A2 molecules or wild type A2 molecules. Each antigen-specific detection molecule was tagged with a unique DNA barcode. Pooled library of 175 antigen specific T-cell detection molecules was stained with 3-5x10⁶ melanoma patient TILs. Comparative results of detection molecules made from Cys-mutant A2 molecules and wild type A2 molecules for all 175 pMHC specificities (x-axis) are shown on the plot. Data plotted on the y-axis are the -log10(P) (in respect to the pMHC-associated DNA barcode) for the DNA-barcoded detection molecules. Dotted line at x = 3 (-log10(0.001)) represent the selected threshold, all readout above this are considered significant for identified antigen specific T-cells. Only Cys-mutant MHC detection molecules (triangles) could identify cancer associated antigen specific T cells in both the melanoma patient samples.

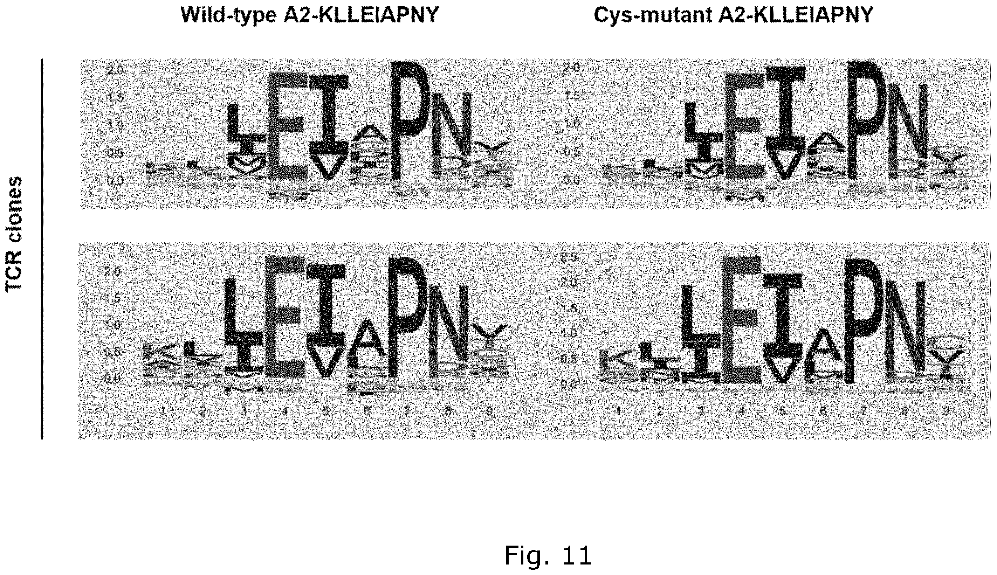

**Figure 11****:** Comparison of the TCR recognition profile of Cys-mutant A2 molecules and wild type A2 molecules. Shannon logos display the amino acid requirements for pMHC-TCR interaction at each position of the peptide. TCR recognition profiles were generated using a DNA barcode-labeled library of 192 peptide-A2 multimers with amino acid variations of the original KLLEIAPNY peptide. TCR recognition for each of these peptide variations was measured with pMHC dextramers prepared from Cys-mutant A2 molecules or from wild type A2 molecules for two T-cell clones that recognized the Merkel Cell Polyomavirus-derived epitope A2-KLLEIAPNY. Shannon logos displaying the TCR fingerprints of the two T-cell clones, using either Cys-mutant A2 molecules or from wild type A2 are depicted.

The present invention will now be described in more detail in the following.

### Detailed description of the invention

### Definitions

Prior to discussing the present invention in further details, the following terms and conventions will first be defined:

### Detection molecule

In the present context, the term "detection molecule" refers to an entity that may be used to detect interaction between an antigenic peptide and a T cell receptor (TCR) or antigenic peptide responsive T cell.

Detection molecules as described herein exist in two forms; (i) a loadable form which comprises one or more MHC class I molecules without any antigenic peptide bound (*i.e.* peptide-free/empty MHC molecules) and (ii) a loaded form which comprises one or more peptide-MHC (pMHC) class I molecules.

Detection molecules comprises also one or more detectable labels that allow detection of interaction between the antigenic peptide provided by the detection molecule and a TCR or antigenic peptide responsive T cell. Thus, detection molecules may *e.g.* be utilized for (i) identifying one or more antigenic peptide responsive T cells in a population of T cells and (ii) TCR fingerprinting by screening of TCR-pMHC interaction of large libraries of antigen-specific detection molecules.

Detection molecules may comprise a connector molecule and/or scaffold molecule to which is attached one or more peptide-free MHC class I molecules and detectable labels. If the detection molecule comprises more than one MHC molecule, it may be designated as a multimer.

In one variant of detection molecules, the connector molecule is streptavidin with four MHC molecules attached, thereby forming a tetramer. Alternatively, the detection molecule may also comprise a polysaccharide scaffold to which are attached MHC molecules either directly or via connector molecules. The polysaccharide may be dextran.

### MHC and Cys-mutant MHC

In the present context, the term "MHC" refers to the major histocompatibility complex, a protein complex whose main function is to bind antigenic peptides derived from pathogens and display them on the cell surface for recognition by the appropriate T-cells.

There are two major classes of MHC molecules, MHC class I molecules and MHC class II molecules. Herein, "MHC" refers to MHC class I molecules. MHC class I molecules consists of an alpha-chain (heavy chain) produced by MHC genes and a beta-chain (light chain or β2-microglubulin) produced by the β2-microglubulin gene.

The heavy chain consists of three domains denoted alpha-1, alpha-2 and alpha-3, respectively. The alpha-1 domain is located next to the non-covalently associated β2-microglubulin. The alpha-3 domain is a transmembrane domain, which anchors the MHC class I molecule in the cell membrane. Together, the alpha-1 and alpha-2 domains forms a heterodimer containing a peptide-binding groove which bind a specific antigenic peptide. The amino acid sequence of the peptide-binding groove is the determinant as to which specific antigenic peptide is bound to the MHC class I molecule.

Naturally, the heavy chain of MHC class I molecules contains four conserved cysteine residues resulting in formation of two disulfide bridges. In the correctly folded conformation of the MHC class I molecule, one disulfide bridge is positioned inside the alpha-2 domain between Cys101 and Cys164, and another disulfide bridge is positioned inside the alpha-3 domain between Cys203 and Cys259, with amino acid numbering referencing to HLA-A without a signal peptide.

In the present invention, an additional disulfide bridge is introduced between the alpha-1 domain and the alpha-2 domain by recombinant introduction of two cysteine residues. Thus, the term "Cys-mutant MHC" is used herein and refers to a disulfide stabilized version of a MHC class I molecule that can be prepared without antigenic peptide bound to the peptide binding cleft of the MHC molecule.

Therefore, the "Cys-mutant MHC" may either be empty or bound to an antigenic peptide. Thus, in the present context there is a distinction between empty MHC molecules free of antigenic peptide (can only be Cys-mutant MHC), and pMHC molecules, which refers to an MHC molecule with bound antigenic peptide (can be either wild type (wt) MHC or Cys-mutant MHC).

In humans, the MHC complex is encoded by the human leukocyte antigen (HLA) gene complex. Thus, in the present context, the term "MHC" encompasses also "HLA". There exist three major types of HLA and therefore MHC in the present context include, but are not limited to, HLA alleles that are coded in the gene loci for HLA-A, HLA-B, and HLA-C. Similarly, MHC include, but are not limited to, MHC class I-like molecules such as HLA-E, HLA-F, HLA-G, HLA-H, MIC A, MIC B, CD1d, ULBP-1, ULBP-2, and ULBP-3.

### Peptide-free MHC class I molecules

In the present context, the term "peptide-free MHC class I molecules" refers to MHC class I molecules to which no antigenic peptide is bound. The terms "peptide-free MHC class I molecule", "empty MHC class I molecule" and "peptide receptive MHC class I molecule" are used interchangeably herein.

### pMHC

In the present context, the term "pMHC" refers to a MHC molecule as defined above to which is bound an antigenic peptide. The term "pMHC" may refer to either wt MHC or Cys-mutant MHC to which is bound antigenic peptide.

### Mutant cysteine residue

In the present context, the term "mutant cysteine residue" refers to a cysteine residues that has artificially been introduced in the heavy chain of a MHC class I molecule. Thus, a mutant cysteine residue is not present in the heavy chain of the corresponding wild type MHC class I molecule.

A mutant cysteine residue may be introduced by mutagenesis techniques known to the person skilled in the art, e.g. site-directed mutagenesis.

### Antigenic peptide

In the present context, the term "antigenic peptide" refers to a peptide that is capable of binding to a major histocompatibility complex (MHC) molecule to form a peptide-MHC (pMHC) complex. The pMHC complex can present the antigenic peptide to immune cells to induce a T-cell receptor dependent immune response. The MHC molecule may be a MHC class I molecule.

In the present context, the phrase "different antigenic peptides" refers to antigenic peptides with non-identical amino acid sequences.

In the present context, the term "target antigenic peptide" refers to an antigenic peptide that is the target of a pharmaceutical candidate. Thus, the pharmaceutical candidate may be, but is not limited to, a TCR, an antigenic peptide responsive T cell, a small molecule drug or a natural compound. To identify any potential off-target issues, libraries of antigenic peptides based on single position mutations of the target antigenic peptide may be generated, and screened for any unintended interaction with the pharmaceutical candidate. These fingerprints of the pharmaceutical candidate may be utilized to assess the risk of adverse effects.

Target antigenic peptides may be, but are not limited to, cancer-associated epitopes, virus epitopes, self-epitopes or other clinically relevant targets.

### Epitope

In the present context, the term "epitope" means the antigenic determinant recognized by the TCR of the T cell or another binding partner, such as a pharmaceutical candidate. The epitope presented by the pMHC is highly specific for any foreign substance and the interaction with the TCR ensures effective expansion and functional stimulation of the specific T cells in a peptide-MHC-directed fashion.

### Library

In the present context, the term "library" refers to a plurality of entities. Thus, a library may be a plurality (or collection) of different antigenic peptides or nucleic acid labels.

### Detectable label

In the present context, the term "detectable label" refers to a moiety detectable by spectroscopic, photochemical, biochemical, immunochemical, or chemical means. The detectable label may generate a measurable signal, such as a fluorescent, radioactive, or chromogenic signal, that can be used to quantitate the amount of bound detectable moiety in a sample. Detectable labels can also be molecular encoded labels, that can be deciphered by sequencing, such as nucleic acid labels.

Detectable labels may be attached to the detection molecule via the MHC molecule or connector molecule either covalently, or through ionic, van der Waals or hydrogen bonds. Examples of detectable labels include, but are not limited to, fluorescent markers, nucleic acid labels, radioisotopes, magnetic markers and metal labels.

In the present context, the phrase "different detectable labels" refers to detectable labels that yield different readouts, *e.g.* fluorescent emissions of different wavelength or nucleic acids of different sequences. Different detectable labels may belong to the same category of labels, *e.g.* two different fluorophores, or may belong to two distinct categories of labels, *e.g.* a radioisotope and a nucleic acid label.

### Sample

In the present context, the term "sample" refers to any solution or solid fraction that comprises a population of T cells. The T cell population may contain T cells with different specificities.

The sample is not limited to any specific source, but may be peripheral blood mononuclear cells, tumors, tissue, bone marrow, biopsies, serum, blood, plasma, saliva, lymph fluid, pleura fluid, cerospinal fluid and synovial fluid. The sample may be extracted from a subject. Samples extracted from individuals may be subjected to the methods described herein to identify and evaluate immune responses during cancer and disease or subsequent to immunotherapy.

### Connector molecule

In the present context, the term "connector molecule" refers to a molecular entity that is part of the detection molecule and to which is attached one or more MHC molecules. A connector molecule may be non-covalently bound to an affinity tag located on a MHC molecule.

Examples of connector molecules include, but are not limited to streptavidin, avidin, metal ion chelates, antibodies *etc.*

The connector molecule may also be attached to a scaffold molecule, such as a polysaccharide like dextran. Thereby, the detection molecules may comprise more than one connector molecules attached to a scaffold molecule, such as at least five connector molecules, such as at least 10 connector molecules, such as at least 15 connector molecules.

### Affinity tag

In the present context, the term "affinity tag" refers to a moiety located on the MHC molecule. An affinity tag binds highly specifically to a connector molecules by non-covalent interaction. Examples of affinity tags include, but are not limited to, biotin, antibody epitopes, His-tags, streptavidin, strep-tactin, polyhistidine, peptides, haptens and metal ion chelates *etc.*

### Non-covalent interaction

In the present context, the term "non-covalent interaction" means any bonding via other interactions than a covalent bond. A non-covalent bond may be formed by e.g. hydrophobic interactions, hydrophilic interactions, ionic interactions, van der walls forces, hydrogen bonding, and combinations thereof.

### Barcode region

In the present context, the term "barcode region" refers to a region that forms part of a nucleic acid label. The barcode region comprises a number of consecutive nucleic that serves as a code that may be used for identifying the detection molecule to which the nucleic acid label is attached upon amplification and/or sequencing.

Each member in a library of nucleic acid labels comprises a unique barcode region with a distinct nucleotide sequence that allows identification of every single member of the nucleic acid label library and the detection molecule to which it is attached. The unique barcode region enables monitoring of interactions between detection molecules and the targets to which they bind, *e.g.* TCRs or antigenic peptide responsive T cells.

The barcode region may vary in length depending on the size of the nucleic acid label library. Thus, the barcode region is not limited to any specific length, but may comprise 5-100 nucleotides, preferably 5-30 nucleotides.

The barcode region can be amplified by PCR. To this end, the barcode region may be flanked by primer regions (forward and reverse) that enables amplification of the barcode region. The primer regions are placed at the 3' end and 5' end of the nucleic acid label, with the barcode region placed in between the primer regions.

### Unique molecular identifier (UMI) region

In the present context, the term "unique molecular identifier (UMI) region" refers to a region of the nucleic acid label which may be used to determine the initial number of oligonucleotide sources of a specific sequence subsequent to amplification. The UMI region is a random sequence of nucleotides that may comprise 2-4, 4-6, 6-8 or 8-10 nucleotide bases. For very large libraries the UMI region may also contain more than 10 nucleotide bases.

### Solid substrate

In the present context, the term "solid substrate" refers to any type of insoluble material to which the detection molecules may be attached. Detection molecules may be covalently or reversibly attached to the solid substrate. Detection molecules that are attached to a solid substrate may be readily separated (by *e.g.* filtration, chromatography, centrifugation, *etc*.) from excess reagents or solvents.

Solid substrates include, but are not limited to, microarrays, glass slides, beads, well plates, particles, filters, gels, tubes, and petri dishes.

A solid substrate comprising immobilized loadable detection molecules may be utilized for fast generation of arrayed antigenic peptide libraries for fingerprinting of pharmaceutical candidates, such as TCRs or antigenic peptide responsive T cells.

### Sequence identity

In the present context, the term "sequence identity" is here defined as the sequence identity between genes or proteins at the nucleotide, base or amino acid level, respectively. Specifically, a DNA and a RNA sequence are considered identical if the transcript of the DNA sequence can be transcribed to the identical RNA sequence.

Thus, in the present context "sequence identity" is a measure of identity between proteins at the amino acid level and a measure of identity between nucleic acids at nucleotide level. The protein sequence identity may be determined by comparing the amino acid sequence in a given position in each sequence when the sequences are aligned. Similarly, the nucleic acid sequence identity may be determined by comparing the nucleotide sequence in a given position in each sequence when the sequences are aligned.

To determine the percent identity of two amino acid sequences or of two nucleic acids, the sequences are aligned for optimal comparison purposes (*e.g*., gaps may be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i.e*., % identity = # of identical positions/total # of positions (*e.g*., overlapping positions) x 100). In one embodiment, the two sequences are the same length.

In another embodiment, the two sequences are of different length and gaps are seen as different positions. One may manually align the sequences and count the number of identical amino acids. Alternatively, alignment of two sequences for the determination of percent identity may be accomplished using a mathematical algorithm. Such an algorithm is incorporated into the NBLAST and XBLAST programs of (Altschul et al. 1990). BLAST nucleotide searches may be performed with the NBLAST program, score = 100, wordlength = 12, to obtain nucleotide sequences homologous to a nucleic acid molecules of the invention. BLAST protein searches may be performed with the XBLAST program, score = 50, wordlength = 3 to obtain amino acid sequences homologous to a protein molecule of the invention.

To obtain gapped alignments for comparison purposes, Gapped BLAST may be utilized. Alternatively, PSI-Blast may be used to perform an iterated search, which detects distant relationships between molecules. When utilizing the NBLAST, XBLAST, and Gapped BLAST programs, the default parameters of the respective programs may be used. See http://www.ncbi.nlm.nih.gov. Alternatively, sequence identity may be calculated after the sequences have been aligned e.g. by the BLAST program in the EMBL database (www.ncbi.nlm.gov/cgi-bin/BLAST). Generally, the default settings with respect to e.g. "scoring matrix" and "gap penalty" may be used for alignment. In the context of the present invention, the BLASTN and PSI BLAST default settings may be advantageous.

The percent identity between two sequences may be determined using techniques similar to those described above, with or without allowing gaps. In calculating percent identity, only exact matches are counted. An embodiment of the present invention thus relates to sequences of the present invention that has some degree of sequence variation.

### Method for flexible, fast and sensitive screening of the interactions of antigenic peptide with TCRs or antigenic peptide responsive T cells

T cell-mediated recognition of peptide-major histocompatibility complex (pMHC) class I and II molecules is crucial for the control of intracellular pathogens and cancer, as well as for stimulation and maintenance of efficient cytotoxic responses. Such interactions may also play a role in the development of autoimmune diseases. Novel insights into this mechanism are crucial to understanding disease development and establishing new treatment strategies. MHC multimers have been used for detection of antigen-responsive T cells since the first report by Altman et al. (1996) showed that tetramerization of pMHC class I molecules provided sufficient stability to T cell receptor (TCR)-pMHC interactions, allowing detection of MHC multimer-binding T cells using flow cytometry. Numerous methods have been developed with the aim of providing a fast reliable platform for screening of pMHC-TCR interactions. Detection molecules founded on MHC multimers and comprising different types of labels have been explored.

However, the persistent challenge of developing technologies based on MHC antigen presentation into truly high-throughput platforms with high sensitivity remains. This is because refolding of MHC molecules is impossible in the absence of antigenic peptide and their production is consequently complex and expensive. Not only are there several thousand MHC class I allotypes, but also a new individualized pMHC multimer must be produced for each antigenic peptide that is to be screened. It would be more efficient and flexible to produce the MHC molecules without antigenic peptides and instead add the antigenic peptide as required just prior to assembling the detection molecules. It would be even more favourable to produce fully assembled detection molecules comprising MHC molecules without antigenic peptides and instead add the antigenic peptide as required.

Thus, to provide a high-throughput method for detection of T cells, the detection molecules of the present invention may be provided with empty MHC class I molecules to which can be added antigenic peptide subsequent to production of the detection molecule. The MHC class I molecules may be stabilized by artificial introduction of a disulfide bridge connecting the alpha-1 and alpha-2 domains of the heavy chain, thereby generating a Cys-mutant MHC variant. The disulfide bridge is positioned outside of the peptide binding groove at the distal end of the C-terminal peptide binding pocket. This structural change of the MHC class I molecule mimics the conformational and dynamic effects of a bound specific antigenic peptide, thereby stabilizing the MHC class I molecule.

Native class I MHC molecules bind peptides of diverse sequences with specific affinity. This is accomplished by the use of conserved amino acids at the ends of peptide-binding groove forming pockets that facilitate peptide binding (typically the A and F pocket of the MHC binding groove). These binding pockets determines a set of requirements for binding of the antigenic peptides, typically referred to as anchor motifs (typically position 2 or 3, and the c-terminal position of the peptide). Although native MHC class I molecules bind many different antigenic peptides through recognition of the anchor motifs, each allele binds only a distinct subset of all available antigenic peptides with high affinity. However, since the artificially introduced disulfide bridge of the Cys-mutant MHC molecules mimics the conformational and dynamic effects of a bound specific antigenic peptide, the detection molecules as described herein can be produced with peptide-free MHC class I molecules that are capable of binding a wide variety of antigenic peptides. These ready-to-use loadable detection molecules, which can be converted to loaded detection molecules comprising antigen-specific pMHC class I molecules in a single-step peptide addition, perfectly align with the state-of-the-art antigen-specific T-cell detection platforms that require the rapid generation of large arrays of MHC multimers.

Moreover, the inventors has found that detection molecules comprising antigenic peptide bound to Cys-mutant MHC molecules are stable over prolonged periods of time and perform better (*i.e.* higher sensitivity) than detection molecules comprising conventional pMHC molecules. Thus, the improved detection molecules will be of great advantage for the fast and effective evaluation of T cell recognition for *e.g.* personalized immunotherapy.

Herein is described detection molecules comprising peptide-free MHC class I molecules that can be assembled with antigenic peptide in one rapid step. The detection molecules may be utilized for improved detection of TCRs or T cells, ultimately enabling identification of even rare and weak interactions. Thus, a first aspect of the present invention relates to a method for detection of one or more antigenic peptide responsive T cells in a sample, the method comprising the following steps:
i. providing loadable detection molecules comprising at least one peptide-free MHC class I molecule and at least one detectable label,
ii. providing at least one antigenic peptide,
iii. contacting the loadable detection molecules with the at least one antigenic peptide to provide loaded detection molecules comprising at least one peptide-MHC (pMHC) class I molecule,
iv. contacting the loaded detection molecules with the sample, and
v. detecting binding of the loaded detection molecules to the one or more antigenic peptide responsive T cells,
wherein each of the at least one antigenic peptide is represented by:
- at least two different detectable labels, or
- at least one detectable label which is a nucleic acid label.

The peptide-free MHC class I molecule may be stabilized by artificial introduction of a disulfide bridge spanning the alpha-1 domain and an alpha-2 domain inside the heavy chain of the MHC class I molecule. Thus, a preferred embodiment of the present invention relates to the method as described herein, wherein the peptide-free MHC class I molecule comprises a heavy chain comprising an alpha-1 domain and an alpha-2 domain connected by a disulfide bridge.

Such modification may be performed by mutagenesis techniques known to the person skilled in the art, *e.g.* site-directed mutagenesis. Specifically, two mutant cysteine residues are introduced in the amino acid sequence of the heavy chain to enable disulfide bridge formation between the alpha-1 and alpha-2 domains. Therefore, an embodiment of the present invention relates to the method as described herein, wherein the disulfide bridge is formed between a mutant cysteine residue positioned in the alpha-1 domain and a mutant cysteine residue positioned in the alpha-2 domain.

Preferably, the position of mutagenesis is selected such that the resulting mutant cysteine residues, placed in the alpha-1 and alpha-2 domains, are within a spatial distance from each other which would under normal protein folding conditions enable formation of a disulfide bridge. Thus, an embodiment of the present invention relates to the method as described herein, wherein the spatial distance between the mutant cysteine residue positioned in the alpha-1 domain and the mutant cysteine residue positioned in the alpha-2 domain is between 2 and 10 angstrom, such as between 2 and 8 angstrom, preferably between 2 and 5 angstrom.

Another embodiment of the present invention relates to the method as described herein, wherein the heavy chain comprises an amino acid sequence selected from:
(a) SEQ ID NO: 1, or
(b) an amino acid sequence having at least 80% sequence identity to the sequence in (a), and
wherein the amino acid sequence comprises a mutant cysteine residue positioned in the alpha-1 domain and a mutant cysteine residue positioned in the alpha-2 domain.

A further embodiment of the present invention relates to the method as described herein, wherein the heavy chain comprises, or consists of, an amino acid sequence selected from:
(a) SEQ ID NO: 1, or
(b) an amino acid sequence having at least 80% sequence identity to the sequence in (a), such as at least 85%, at least 90%, at least 95%, at least 96%, at least 97%, at least 98%, or at least 99% sequence identity to the sequence in (a), and
wherein said amino acid sequence comprises a mutant cysteine residue positioned in the alpha-1 domain and a mutant cysteine residue positioned in the alpha-2 domain.

Preferred mutations of the two cysteine residues include the modification of the amino acid in position 139 and either of the amino acids in positions 84 or 85. The amino acid in position 139 is positioned in the alpha-2 domain of the heavy chain and is in many cases an alanine residue. The amino acid in position 84 or 85 is positioned in the alpha-1 domain of the heavy chain and is in many cases a tyrosine residue. The cysteine mutations may be introduced by amino acid substitution through modification of the gene sequence of the heavy chain using standard genetic engineering. Therefore, a preferred embodiment of the present invention relates to the method as described herein, wherein the mutant cysteine residue in the alpha-1 domain is at amino acid residue 84 or 85 and the mutant cysteine residue positioned in the alpha-2 domain is at amino acid residue 139.

Upon folding of the MHC class I molecule, a disulfide bridge is formed between Cys-84 or Cys-85, and Cys-139. The newly formed disulfide bridge stabilizes the MHC class I molecule so that it remains stable in solution in absence of an antigenic peptide. The introduction of cysteine mutant residues may be applied to any type of MHC class I molecule. Thus, an embodiment of the present invention relates to the method as described herein, wherein the at least one peptide-free MHC class I molecule is a vertebrate peptide-free MHC class I molecule, such as a human, murine, rat, porcine, bovine or avian molecule. Another embodiment of the present invention relates to the method as described herein, wherein the peptide-free MHC class I molecule is a human molecule. A further embodiment of the present invention relates to the method as described herein, wherein the peptide-free MHC class I molecule is based on a MCH molecule selected from the group consisting of HLA-A, HLA-B, HLA-C, HLA-E, HLA-F, HLA-G, HLA-H, MIC A, MIC B, CD1d, ULBP-1, ULBP-2, and ULBP-3.

The method as described herein is also applicable for use with a peptide-free MHC molecule originating from a mouse. This mouse MHC molecule is designated the H-2 complex. Thus, an embodiment of the present invention relates to the method as described herein, wherein the heavy chain is a H-2 molecule, such as H-2Kb or H-2Db. Another embodiment of the present invention relates to the method as described herein, wherein the heavy chain comprises an amino acid sequence selected from:
(a) any one of SEQ ID NO: 12 or SEQ ID NO: 13, or
(b) an amino acid sequence having at least 80% sequence identity to any one of the sequences in (a).

Preferred variants of peptide-free MHC class I molecules includes HLA-A, HLA-B and H-2, each comprising two mutant cysteine residues. Thus, an embodiment of the present invention relates to the method as described herein, wherein the heavy chain comprises an amino acid sequence selected from:
(a) any one of SEQ ID NO: 2-13, or
(b) an amino acid sequence having at least 80% sequence identity to any one of the sequences in (a), and
wherein the amino acid sequence comprises a mutant cysteine residue positioned in the alpha-1 domain and a mutant cysteine residue positioned in the alpha-2 domain.

Another embodiment of the present invention relates to the method as described herein, wherein the heavy chain comprises, or consists of, an amino acid sequence selected from:
(a) any one of SEQ ID NO: 2-13, or
(b) an amino acid sequence having at least 80% sequence identity to any one of the sequences in (a), such as at least 85%, at least 90%, at least 95%, at least 96%, at least 97%, at least 98%, or at least 99% sequence identity to any one of the sequences in (a), and
wherein the amino acid sequence comprises a mutant cysteine residue positioned in the alpha-1 domain and a mutant cysteine residue positioned in the alpha-2 domain.

It is noted that the phrasing "SEQ ID NO: 2-13" is to be understood as "SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, or SEQ ID NO:13".

A further embodiment of the present invention relates to the method as described herein, wherein the heavy chain comprises an amino acid sequence selected from:
(a) any one of SEQ ID NO: 2-11, or
(b) an amino acid sequence having at least 80% sequence identity to any one of the sequences in (a).

A yet further embodiment of the present invention relates to the method as described herein, wherein the heavy chain comprises an amino acid sequence selected from:
(a) any one of SEQ ID NO: 2-6, or
(b) an amino acid sequence having at least 80% sequence identity to any one of the sequences in (a).

A much utilized allele of HLA for T cell detection is the HLA-A 02:01 allele. Therefore, a preferred embodiment of the present invention relates to the method as described herein, wherein the heavy chain comprises an amino acid sequence selected from:
(a) SEQ ID NO: 2, or
(b) an amino acid sequence having at least 80% sequence identity to the sequence in (a).

Another embodiment of the present invention relates to the method as described herein, wherein the heavy chain comprises, or consists of, an amino acid sequence selected from:
(a) SEQ ID NO: 2, or
(b) an amino acid sequence having at least 80% sequence identity to the sequence in (a), such as at least 85%, at least 90%, at least 95%, at least 96%, at least 97%, at least 98%, or at least 99% sequence identity to the sequence in (a).

Naturally, the heavy chain is associated with a β2-microglobulin molecule (B2M) to form a MHC class I molecule. Specifically, the alpha-3 domain of the heavy chain is positioned adjacent to B2M. Thus, an embodiment of the present invention relates to the method as described herein, wherein the at least one peptide-free MHC class I molecule comprises a β2-microglobulin molecule.

Another embodiment of the present invention relates to the method as described herein, wherein the β2-microglobulin molecule comprises an amino acid sequence selected from:
(a) SEQ ID NO: 15, or
(b) an amino acid sequence having at least 80% sequence identity to the sequence in (a).

The peptide-free MHC class I molecule may also be provided as a finalized fusion protein with B2M connected to the heavy chain via a linker. The linker is preferably a peptide linker. Thus, an embodiment of the present invention relates to the method as described herein, wherein the at least one peptide-free MHC class I molecule comprises an amino acid sequence selected from:
(a) SEQ ID NO: 16, or
(b) an amino acid sequence having at least 80% sequence identity to the sequence in (a).

The detection molecule of the present invention may be provided as complex molecules further comprising connector molecules suitable for assembling e.g. several peptide-free MHC class I molecule within a single detection molecule. Thus, an embodiment of the present invention relates to the method as described herein, wherein the loadable detection molecules comprises at least one connector molecule, at least one peptide-free MHC class I molecule and at least one detectable label. Another embodiment of the present invention relates to the method as described herein, wherein the at least one peptide-free MHC class I molecule is attached to a connector molecule.

The connector molecules provide a flexible template to which affinity-tagged peptide-free MHC class I molecules may be fixed in a modular fashion. Affinity tags are molecular species that bind specifically to the connector molecules through, but not limited to, non-covalent interactions. By attaching an affinity tag to each peptide-free MHC class I molecule, it is therefore easy to assemble a custom-built loadable detection molecule. Thus, a preferred embodiment of the present invention relates to the method as described herein, wherein the peptide-free MHC class I molecule comprises an affinity tag.

Another embodiment of the present invention relates to the method as described herein, wherein the peptide-free MHC class I molecule is attached to the connector molecule via non-covalent interactions between the connector molecule and the affinity tag on the peptide-free MHC class I molecule.

Many known compatible pairs of affinity tags and connector molecules may be used with the present invention and include, but are not limited to, biotin/streptavidin, biotin/avidin, biotin/neutravidin, biotin/strep-tactin, poly-His/metal ion chelate, peptide/antibody, glutathione-S-transferase/glutathione, epitope/antibody, maltose binding protein/amylase and maltose binding protein/maltose. Other known compatible pairs of affinity tags and connector molecules may also be used with the present invention. An embodiment of the present invention relates to the method as described herein, wherein the affinity tag is selected from the group consisting of biotin, antibody epitopes, His-tags, streptavidin, strep-tactin, polyhistidine, peptides, haptens and metal ion chelates. Another embodiment of the present invention relates to the method as described herein, wherein the connector molecule is selected from the group consisting of streptavidin, avidin, metal ion chelates and antibodies. A preferred embodiment of the present invention relates to the method as described herein, wherein the connector molecule is streptavidin and the affinity tag is biotin.

The affinity tag, such as biotin, is placed on the MHC class I molecule. The attachment of biotin to the heavy chain may be accomplished by inclusion of a handle, such as an Avi-tag, for biotinylation of the heavy chain. Therefore, an embodiment of the present invention relates to the method as described herein, wherein the heavy chain comprises an Avi-tag. Another embodiment of the present invention relates to the method as described herein, wherein the heavy chain comprises an amino acid sequence selected from:
(a) SEQ ID NO: 14, or
(b) an amino acid sequence having at least 80% sequence identity to the sequence in (a).

In a variant of the present invention, the detection molecules comprises a scaffold molecule to which connector molecules or peptide-free MHC class I molecules may be bound. Typically, in this variant the peptide-free MHC class I molecules would be attached to the connector molecules, which in turn is attached to the scaffold molecule. These attachments may be either covalent or non-covalent attachment, preferably non-covalent attachments. The scaffold molecule may be, but are not limited to, polysaccharides, such as dextran. Thus, an embodiment of the present invention relates to the method as described herein, wherein the loadable detection molecules comprises a scaffold molecule to which is attached at least one connector molecule comprising at least one peptide-free MHC class I molecules and at least one detectable label. Another embodiment of the present invention relates to the method as described herein, wherein the scaffold molecule is dextran and the connector molecule is streptavidin.

Examples of other suitable scaffolds molecules include, but is not limited to, polysaccharides, synthetic polysaccharides, vinyl polymers, poly ethylene glycol, poly propylene glycol, derivatised cellulosics, strep-tactin and poly-streptavidin.

The loadable detection molecules may be provided with more than one peptide-free MHC class I molecule in order to ensure sufficient binding of detection molecules to antigenic peptide responsive T cells in a sample. MHC multimers has been used extensively for the last couple of decades and especially MHC tetramers are regarded as the state-of-the-art within T cell detection techniques. Thus, an embodiment of the present invention relates to the method as described herein, wherein the loadable detection molecule comprises at least two peptide-free MHC class I molecules, such at least three peptide-free MHC class I molecules, preferably four peptide-free MHC class I molecules. A preferred embodiment of the present invention relates to the method as described herein, wherein the loadable detection molecule comprises four peptide-free MHC class I molecules attached to streptavidin via non-covalent interactions between streptavidin and a biotin tag on each peptide-free MHC class I molecule.

The loadable detection molecules comprise at least one detectable label, which may be attached to the connector molecule, the peptide-free MHC class I molecule or the scaffold molecule. Preferably, the detectable label is attached to the connector molecule. Therefore, an embodiment of the present invention relates to the method as described herein, wherein the at least one detectable label is attached to the connector molecule. Attachment of the detectable label may be either covalent or non-covalent.

The number of detectable labels for each detection molecule may vary depending on the mode of detection. For detection wherein each of the at least one antigenic peptide is represented by at least two different detectable labels, each detection molecule may comprise only a single detectable label, such as a fluorescent marker or radioisotope. Thus, an embodiment of the present invention relates to the method as described herein, wherein the detection molecule comprises a single detectable label. For detection wherein each of the at least one antigenic peptide is represented by at least one detectable label which is a nucleic acid label, each detection molecule may in addition to said nucleic acid label comprise additional detectable labels. Thus, an embodiment of the present invention relates to the method as described herein, wherein the detection molecule comprises a nucleic acid label and at least one detectable label. A preferred embodiment of the present invention relates to the method as described herein, wherein the detection molecule comprises a nucleic acid label and at least one fluorescent marker, preferably one fluorescent marker.

Because the loadable detection molecules are assembled without bound antigenic peptide, they are perfectly suited for rapid generation of large libraries on-demand immediately prior to use. Depending on the screening setup, different numbers of antigenic peptides may be desired in the assay. In some assays it may be preferred to only screen for a small amount of very specific types of T cells, whereas other assays may aim at identifying a large selection of different T cells in parallel. Thus, an embodiment of the present invention relates to the method as described herein, wherein at least two different antigenic peptides are provided in step ii), such as at least three, such as at least four, such as at least five, such as at least six, such as at least seven, such as at least eight, such as at least nine, such as at least ten, such as at least fifteen, such as at least twenty, such as at least twenty-five, such as at least fifty, such as at least hundred different antigenic peptides. However, the method utilizing the loadable detection molecules are also suitable for larger libraries. Thus, another embodiment of the present invention relates to the method as described herein, wherein at least 1.000 different antigenic peptides are provided in step ii), such as 10.000, such 100.000, such as 1.000.000 different antigenic peptides.

For the purpose of simultaneous high-throughput screening for many different antigenic peptides, it is to be understood that a plurality of different loaded detection molecules are generated and contacted with the sample. Thus, an embodiment of the present invention relates to the method as described herein, wherein a plurality of loadable detection molecules are provided. Another embodiment of the present invention relates to the method as described herein, wherein the sample is contacted with a plurality or library of different loaded detection molecules.

The antigenic peptides presented by the pMHC molecules ultimately decides which type of T cells will be identified by the method described herein. The antigenic peptides suitable for use with the aAPC scaffold according to the present invention may essentially come from any source. The antigenic source may include, but is not limited to, a human, a virus, a bacterium, a parasite, a plant, a fungus, or a tumor. Thus, an embodiment of the present invention relates to the method as described herein, wherein the antigenic peptide of the pMHC molecule is derived from a source selected from the group consisting of a human, a virus, a bacterium, a parasite, a plant, a fungus, and a tumor.

The antigenic peptide may be an already known immunogenic epitope of *e.g.* a virus or a tumor cell, thus enabling detection of the presence of T cells responsive to this antigen and the subsequent diagnosis of a viral infection or cancer. The antigenic peptide may also be an unknown epitope, with the detection of T cells responsive to this epitope being indicative for the presence of an immunogenic amino acid sequence within this peptide, thus enabling the identification of immunogenic regions or epitopes in *e.g.* a polypeptide.

In a variant of the method described herein, following the combicoding approach, the loadable detection molecules are provided with at least one detectable label and the antigen is represented, or encoded, by at least two differently labelled detection molecules. Thus, each individual antigenic peptide is loaded on at least two distinct detection molecules each comprising a different detectable label, such as different fluorescent markers or radioisotopes.

The loadable detection molecules may be provided with at least two different detectable labels, such as three, four, five, six, seven or eight different detectable labels. Thus, the antigenic peptide may be represented by one multiple-labelled detection molecule. In this variant of the method, each antigenic peptide is loaded onto a distinct detection molecule comprising two different detectable labels, such as different fluorescent markers.

Thus, an embodiment of the present invention relates to the method as described herein, wherein the at least one antigenic peptide is represented by at least two different detectable labels, and binding of the loaded detection molecules to the one or more antigenic peptide responsive T cells is detected by detecting the presence of the at least two different detectable labels bound to an antigen responsive T cell through the loaded detection molecules. A preferred embodiment of the present invention relates to the method as described herein, wherein each individual antigenic peptide is loaded on at least two distinct detection molecules each comprising a different detectable label. Another preferred embodiment of the present invention relates to the method as described herein, wherein each antigenic peptide is loaded onto a distinct detection molecule comprising two different detectable labels.

Interactions between the detection molecules and antigenic peptide responsive T cells is detected by a readout generated by the detectable label. In principle any type of detectable label may be used for the method. Thus, the method as described herein may be applied using a selection of different types of detectable label, either alone or in combination with each other. Therefore, an embodiment of the present invention relates to the method as described herein, wherein the at least two different detectable labels are selected from the group consisting of fluorescent markers, radioisotopes, magnetic markers and metal labels. This could be e.g. two different fluorescent labels or a fluorescent marker in combination with a metal label.

Fluorescent markers constitutes a specially preferred detectable label due to the broad availability of fluorescent markers and the widespread familiarity with fluorescent measurements in most laboratories. Thus, an embodiment of the present invention relates to the method as described herein, wherein the at least two different detectable labels are fluorescent markers. Of practical importance to the method is that the fluorescent markers may be excited by laser lines found in common flow cytometers. Thus, preferred are fluorescent markers that can be excited by the UV laser (355nm), the violet laser (405nm), the blue laser (488nm), the yellow/green laser (561nm) or the red laser (640nm). Therefore, an embodiment of the present invention relates to the method as described herein, wherein the fluorescent markers are one or more fluorescent markers excited at a wavelength selected from the group consisting of 355 nm, 405 nm, 488 nm, 561 nm and 640 nm. Examples of suitable fluorescent markers include, but are not limited to PE, BV421, BUV395, APC-Cy^{™}7, APC, BV510, BV480, PE-Cy^{™}5, PE-CF594, PE-Cy^{™}7, PerCP-Cy^{™}5.5, PerCP, FITC, BV605, BUV737, BV650, V500, BV786, BUV496, BUV805, V450, APC-R700, BUV563, BV711, BB515 and BUV661.

Alternative fluorescent markers, such as quantum dots (QDots) may also be utilized for the method as described herein.

Another group of preferred detectable labels are metal labels, which constitutes a familiar and favorable group of labels. Mass cytometry is a mass spectrometry technique based on inductively coupled plasma mass spectrometry and time of flight mass spectrometry used for the determination of the properties of cells (cytometry). In this approach, antibodies and detection molecules, such as MHC tetramers, are conjugated with isotopically pure metal elements, and these antibodies and MHC tetramers are used to label cellular proteins on *e.g.* T cells.

The cells are nebulized and sent through an argon plasma, which ionizes the metal-conjugated antibodies and tetramers. The metal signals are then analyzed by a time-of-flight mass spectrometer. The approach overcomes potential limitations of spectral overlap in flow cytometry by utilizing discrete isotopes as a reporter system instead of traditional fluorophores which may have broad emission spectra.

CyTOF (cytometry by time of flight) has been commercialized and a variety of commonly used metal-antibody conjugates are available. Advantages are minimal overlap in metal signals and the instrument is theoretically capable of detecting more than 100 parameters per cell. However, current methods based on single metal labelling limits cytometer use to around 40 parameters per cell. Combining two or more metal labels on a detection molecule, such as an MHC tetramer, dramatically increase the number of T cells specificities that can be analyzed within a sample. Thus, an embodiment of the present invention relates to the method as described herein, wherein the at least two different detectable labels are metal labels.

For detection of larger pools of different TCRs or antigenic peptide responsive T cell using the combicoding approach, it is necessary to introduce an increasing number of different detectable labels in the method. Thus, an embodiment of the present invention relates to the method as described herein, wherein the number of different detectable labels is at least three, such as at least four, such as at least five, such as at least six, such as at least seven, such as at least eight, such as at least nine, such as at least ten, such as at least fifteen, such as at least twenty, such as at least fifty.

Another means of increasing the number of antigenic peptide responsive T cells that may be detected in parallel is to let each antigenic peptide be represented by more than two different detectable labels. Therefore, an embodiment of the present invention relates to the method as described herein, wherein each antigenic peptide is represented by at least three or at least four different labels. Another embodiment of the present invention relates to the method as described herein, wherein each detection molecules comprises at least three or at least four different labels.

The mode of detection according to the method as described herein depends on the choice of detectable label. The person skilled in the art would typically know which detection means to pair with a given detectable label. An embodiment of the present invention relates to the method as described herein, wherein detection comprises one or more methods selected from the group consisting of flow cytometry, fluorescence spectrometry and mass cytometry. A preferred embodiment of the present invention relates to the method as described herein, wherein the detectable labels are fluorescent markers and the mode of detection is flow cytometry.

An estimated 100,000-750,000 pMHC class I complexes are expressed for each allelic product (for human leukocyte antigen (HLA)-A and HLA-B loci), and each individual carries ~10⁷ different TCRs, each detecting up to 10⁶ variations of a given peptide sequence. Thus, another variant of the method described herein follow the barcoding approach to expand the size of the assessable libraries of antigenic peptides and increase the analytical range within which TCRs or antigenic peptide responsive T cells may be detected.

Barcodes are labels that uniquely identify the entity to which it has been attached. Nucleic acids are a preferred building block for barcodes due to the superior combinatorial possibilities permitting generation of large reservoirs of unique labels. A nucleic acid label may comprise a unique region of consecutive nucleotides (barcode region) that allows identification of the label, and primer regions that allows amplification of the label. Moreover, nucleic acid labels may comprise a region that may be used to estimate the initial number of oligonucleotide sources of a specific sequence. Therefore, an embodiment of the present invention relates to the method as described herein, wherein the at least one detectable label is a nucleic acid label comprising
i. a 5'first primer region (forward), a barcode region and a 3' second primer region (reverse), and
ii. a unique molecular identifier (UMI) region of random nucleotide bases
wherein the barcode region is a unique barcode serving as an identification tag for the detection molecule.

Another embodiment of the present invention relates to the method as described herein, wherein the nucleic acid label is selected from the group consisting of a DNA label, a RNA label and an artificial nucleic acid label. A preferred embodiment of the present invention relates to the method as described herein, wherein the nucleic acid label is a DNA label.

An important consideration when quantifying amplified oligonucleotide material is whether the resulting amount of oligonucleotide material originates from a single oligonucleotide source or from several identical oligonucleotide sources. To overcome this problem, small regions called unique molecular identifiers (UMI) may be added to the oligonucleotide material. These UMI regions are random sequences that subsequent to amplification may be used to determine the initial number of oligonucleotide sources of a specific sequence. Thus, an embodiment of the present invention relates to the method as described herein, wherein the nucleic acid label comprises a unique molecular identifier (UMI) region of random nucleotide bases. Such a UMI region may contain 2-4, 4-6, 6-8 or 8-10 nucleotide bases. For very large libraries the UMI region may also contain more than 10 nucleotide bases. Thus, an embodiment of the present invention relates to the method as described herein, wherein the UMI region contains 6-8 random nucleotide bases.

The design of the nucleic acid label may be further revised by addition of one or more additional elements. Therefore, an embodiment of the present invention relates to the method as described herein, wherein the nucleic acid label further comprises one or more selected from the group consisting of linker molecules, adaptors for sequencing, and annealing regions. Amongst others, two designs are preferred; a single oligo nucleic acid label, and a dual oligo nucleic acid label. The single oligo nucleic acid label comprises a forward primer, a barcode region, a reverse primer and a UMI region. The dual oligo nucleic acid label consists of two sequences, the first comprising a forward primer region, a UMI region, a first barcode region, and an annealing region, and the second comprising a complementary annealing region, a second barcode region, a UMI region and a reverse primer region. The first sequence of the dual oligo nucleic acid label may be biotinylated. In Example 6, the first sequence is referred to as oligo A, the second sequence is referred to as oligo B, the first barcode is referred to as barcode A, and the second barcode is referred to as barcode B. Following annealing of the first sequence with the second sequence and before the attachment to the detection molecule, the first and second sequences are elongated to obtain their double-stranded form. After the isolation of detection molecule-binding T cells, the DNA barcodes are amplified by PCR and analyzed by sequencing. Thus, an embodiment of the present invention relates to the method as described herein, wherein the nucleic acid label comprises two sequences, the first comprising a forward primer region, a UMI region, a barcode region, and an annealing region, and the second comprising a complementary annealing region, a barcode region, a UMI region and a reverse primer region.

The barcode labelled detection molecules binding to T cells may initially be sorted using a fluorescent marker. Thus, an embodiment of the present invention relates to the method as described herein, wherein the loadable detection molecule comprises a nucleic acid label and a fluorescent marker. Detection molecules binding to a T cell may thus be fluorescently sorted, e.g. by fluorescence-activated cell sorting (FACS), and subsequently the composition of the associated nucleic acid labels can be retrieved by nucleic acid amplification and high-throughput sequencing.

Nucleic acid labels are suited for identifying individual members of large libraries of detection molecules. Therefore, an embodiment of the present invention relates to the method as described herein, wherein at least 25 different antigenic peptides are provided in step ii, such as at least 50, such as at least 100, such as at least 500, such as at least 1.000, such as at least 10.000, such as at least 100.000, such as at least 1.000.000. Another embodiment of the present invention relates to the method as described herein, wherein detection comprises one or more methods selected from the group consisting of amplification of the label, sequencing of the label, DNA sequencing, mass spectrometry, gel electrophoresis, gel filtration, PAGE, column fractionation, PCR, and QPCR. Preferably, detection of detection molecules comprising DNA labels is performed by DNA sequencing.

The method as described herein may comprise one or more additional steps for improving performance. Therefore an embodiment of the present invention relates to the method as described herein, wherein the method comprises one or more additional steps selected from the group consisting of incubation, washing, purification and isolation. The additional steps may be applied to the method following either the combicoding approach or the barcoding approach. A preferred embodiment of the present invention relates to the method as described herein, wherein the step of isolation comprises one or more methods selected from the group consisting of flow cytometry, FACS sorting, washing, centrifugation, precipitation, filtration, affinity column, and immobilization.

The method is not limited to a specific type of sample, but may be applied to any solution or solid fraction that comprises a population of T cells. The T cell population may contain T cells with different specificities. The origin of the sample is not restricted to any specific species, but human samples are preferred. An embodiment of the present invention relates to the method as described herein, wherein the sample is selected from the group consisting of peripheral blood mononuclear cells, tumors, tissue, bone marrow, biopsies, serum, blood, plasma, saliva, lymph fluid, pleura fluid, cerospinal fluid and synovial fluid. Another embodiment of the present invention relates to the method as described herein, wherein the T cells are selected from the group consisting of CD8 T cells, CD4 T cells, regulatory T cells, natural killer T (NKT) cells, gamma-delta T cells, NK cells and innate mucosal-associated invariant T (MAIT) cells.

The Cys-mutant MHC class I molecules may be loaded with any type of antigenic peptide and as such the method is not limited to any specific type or variant of antigenic peptides. However, some groups of antigenic peptides harness more interest than others due to their potential therapeutic or diagnostic applications. In cancer immunotherapy, there is a great interest in understanding T-cell recognition of cancer-associated epitopes as well as mutation-derived neoepitopes and the potential to explore these for therapeutic strategies. In most cases, for the comprehensive evaluation of neoepitope recognition, screening of large pMHC libraries is necessary. The ability to apply loadable detection molecules will ease the process of generating such large pMHC libraries. Therefore, an embodiment of the present invention relates to the method as described herein, wherein the antigenic peptides are selected from cancer-associated epitopes, virus epitopes, self-epitopes and variants thereof. Another embodiment of the present invention relates to the method as described herein, wherein the cancer-associated epitopes are virus epitopes associated with a virus-induced cancer. A preferred embodiment of the present invention relates to the method as described herein, wherein the virus epitopes are from a virus selected from the group consisting of human papillomavirus (HPV), Merkel cell polyomavirus (MCV), cytomegalovirus (CMV), Epstein-Barr virus (EBV), human T-lymphotropic virus (HTLV), hepatitis B virus (HBV), hepatitis C virus (HCV) and influenza virus. A further embodiment of the present invention relates to the method as described herein, wherein the antigenic peptides are neoepitopes.

The Cys-mutant MHC class I molecules as described herein are an efficient tool to produce quality-assured reagents (e.g. detection molecules) for immune monitoring, for diagnostics, and in therapeutic applications such as the isolation or specific stimulation of antigen-specific T cells for adaptive T-cell transfer in immunotherapy approaches. As demonstrated herein, detection molecules comprising Cys-mutant MHC class I molecules provide an upgrade over detection molecules comprising wt MHC Class I molecules in respect of flexibility, stability and sensitivity. The detection molecules comprising Cys-mutant MHC class I molecules show a higher fluorescence intensity and better separation from detection molecule-negative T cells than detection molecules comprising wt MHC class I molecules, resulting in an increased staining index. Without being bound by theory, the enhanced stability of the loaded detection molecules comprising Cys-mutant MHC class I molecules may impact the staining capacity. Thus, an embodiment of the present invention relates to the method as described herein, wherein the loadable detection molecules comprising at least one peptide-free MHC class I molecule increase the staining index as compared to detection molecules comprising wt MHC class I molecules. The staining index is calculated as (Median of Positive - Median of Negative) / (SD of Negative * 2) based on the fluorescent output during detection.

Another aspect of the present invention relates to a method for detection of one or more antigenic peptide responsive T cells in a sample, the method comprising the following steps:
i. providing loadable detection molecules comprising at least one peptide-free MHC class I molecule and at least one detectable label,
ii. providing at least one antigenic peptide,
iii. contacting the loadable detection molecules with the at least one antigenic peptide to provide loaded detection molecules comprising at least one peptide-MHC (pMHC) class I molecule,
iv. contacting the loaded detection molecules with the sample, and
v. detecting binding of the loaded detection molecules to the one or more antigenic peptide responsive T cells,
wherein each of the at least one antigenic peptide is represented by at least two different detectable labels. All embodiments and features described in the context of any one of the other aspects apply also to this specific aspect.

Another embodiment of the present invention relates to the method as described herein, wherein binding of the detection molecules to the one or more antigenic peptide responsive T cells is detected by detecting the at least two different detectable labels.

A further aspect of the present invention relates to a method for detection of one or more antigenic peptide responsive T cells in a sample, the method comprising the following steps:
i. providing loadable detection molecules comprising at least one peptide-free MHC class I molecule and at least one nucleic acid label,
ii. providing at least one antigenic peptide,
iii. contacting the loadable detection molecules with the at least one antigenic peptide to provide loaded detection molecules comprising at least one peptide-MHC (pMHC) class I molecule,
iv. contacting the loaded detection molecules with the sample, and
v. detecting binding of the loaded detection molecules to the one or more antigenic peptide responsive T cells.

All embodiments and features described in the context of any one of the other aspects apply also to this specific aspect.

A preferred embodiment of the present invention relates to the method as described herein, wherein the method comprises an additional step (vi) of determining the identity of the at least one pMHC class I molecule. More explicitly an embodiment of the present invention relates to a method for detection of one or more antigenic peptide responsive T cells in a sample, the method comprising the following steps:
i. providing loadable detection molecules comprising at least one peptide-free MHC class I molecule and at least one nucleic acid label,
ii. providing at least one antigenic peptide,
iii. contacting the loadable detection molecules with the at least one antigenic peptide to provide loaded detection molecules comprising at least one peptide-MHC (pMHC) class I molecule,
iv. contacting the loaded detection molecules with the sample,
v. detecting binding of the loaded detection molecules to the one or more antigenic peptide responsive T cells, and
vi. determining the identity of the at least one pMHC class I molecule.

Loadable detection molecules comprising at least one peptide-free MHC class I molecules and a nucleic acid label may be used to detect T cells out of a sample or for measuring the affinity-based TCR-pMHC interaction and consequently providing the TCR's recognition preferences. Thus, another aspect of the present invention relates to a loadable detection molecule comprising at least one peptide-free MHC class I molecule and a nucleic acid label. All embodiments and features described in the context of any one of the other aspects apply also to this specific aspect.

An embodiment of the present invention relates to the loadable detection molecule as described herein, wherein the peptide-free MHC class I molecule is as defined herein. Another embodiment of the present invention relates to the loadable detection molecule as described herein, wherein the peptide-free MHC class I molecule comprises a heavy chain comprising an alpha-1 domain and an alpha-2 domain connected by a disulfide bridge.

A preferred embodiment of the present invention relates to the loadable detection molecule as described herein, wherein the heavy chain comprises, or consists of, an amino acid sequence selected from:
(a) SEQ ID NO: 1, or
(b) an amino acid sequence having at least 80% sequence identity to the sequence in (a), such as at least 85%, at least 90%, at least 95%, at least 96%, at least 97%, at least 98%, or at least 99% sequence identity to the sequence in (a), and
wherein said amino acid sequence comprises a mutant cysteine residue positioned in the alpha-1 domain and a mutant cysteine residue positioned in the alpha-2 domain.

A further embodiment of the present invention relates to the loadable detection molecule as described herein, wherein the mutant cysteine residue in the alpha-1 domain is at amino acid residue 84 or 85 and the mutant cysteine residue positioned in the alpha-2 domain is at amino acid residue 139.

An even further embodiment of the present invention relates to the loadable detection molecule as described herein, wherein the heavy chain comprises an amino acid sequence selected from:
(a) any one of SEQ ID NO: 2-13, or
(b) an amino acid sequence having at least 80% sequence identity to the sequence in (a), such as at least 85%, at least 90%, at least 95%, at least 96%, at least 97%, at least 98%, or at least 99% sequence identity to the sequence in (a), and
wherein the amino acid sequence comprises a mutant cysteine residue positioned in the alpha-1 domain and a mutant cysteine residue positioned in the alpha-2 domain.

The nucleic acid label of the loadable detection molecule may be as defined previously herein. An embodiment of the present invention relates to the loadable detection molecule as described herein, wherein the nucleic acid label is as defined as described herein. A preferred embodiment of the present invention relates to the loadable detection molecule comprising at least one peptide-free MHC class I molecule and a nucleic acid label, wherein the at least one detectable label is a nucleic acid label comprising
i. a 5'first primer region (forward), a barcode region and a 3' second primer region (reverse), and
ii. a unique molecular identifier (UMI) region of random nucleotide bases
wherein the barcode region is a unique barcode serving as an identification tag for the detection molecule.

The loadable detection molecule may comprise a connector molecule, such as streptavidin, and/or scaffold molecule, such as dextran, to which is attached one or more peptide-free MHC class I molecules and detectable labels. A preferred embodiment of the present invention relates to the loadable detection molecule as described herein, wherein the loadable detection molecule comprises four peptide-free MHC class I molecules attached to streptavidin via non-covalent interactions between streptavidin and a biotin tag on each peptide-free MHC class I molecule.

The loadable detection molecules may be present either in solution or immobilized on a solid substrate. By immobilizing detection molecules on a solid support, it is possible to easily separate loaded detection molecules bound to TCRs or antigenic peptide responsive T cells from any residual and unbound reagents and/or exchange buffers during washing procedures etc. Moreover, by spotting loadable detection molecules in pre-defined patterns on the solid substrate it is possible to provide advanced arrays with known coordinates for screening purposes. Thus, an embodiment of the present invention relates to the loadable detection molecule as described herein, wherein the loadable detection molecule is immobilized on a solid substrate.

The solid substrate may be of a variety of materials depending on the setting in which the loadable detection molecule is to be used. Microarrays of loadable detection molecules may be advantageous for screening of large libraries, while loadable detection molecules immobilized on beads may be advantageous in purification protocols. Thus, an embodiment of the present invention relates to the loadable detection molecule as described herein, wherein the solid substrate is selected from the group consisting of microarrays, glass slides, beads, well plates, particles, filters, gels, tubes, and petri dishes. A preferred embodiment of the present invention relates to the loadable detection molecule as described herein, wherein the solid substrate is a microarray.

An additional aspect of the invention relates to a solid substrate comprising at least one peptide-free MHC class I molecule. The peptide-free MHC class I molecule may be as defined herein, such as comprising a heavy chain comprising an alpha-1 domain and an alpha-2 domain connected by a disulfide bridge.

The loadable detection molecules may also be provided as a composition suitable for use with either the combicoding approach or the barcoding approach as described herein. Thus, an aspect of the present invention relates to a composition for detection of one or more antigenic peptide responsive T cells in a sample, the composition comprising:
(a) at least two loadable detection molecules as described herein, or
(b) at least three loadable detection molecules comprising at least one peptide-free MHC class I molecule as defined herein and at least one detectable label, wherein each loadable detection molecule comprises a different detectable label.

Another aspect of the present invention relates to use of a loadable detection molecule as described herein or a composition as described herein for detection of one or more antigenic peptide responsive T cells in a sample.

Loadable detection molecules or compositions thereof may be provided as ready-to-use packages/kits that comprises loadable detection molecules and antigenic peptides of interest for easy and rapid on demand formation of loaded detection molecules. The kits may be provided with libraries of antigenic peptides of various sizes, spanning *e.g.* from few very specific antigenic peptides of importance to a given disease to an antigenic peptide library of the human peptidome. Therefore, another aspect of the present invention relates to a kit for detection of one or more antigenic peptide responsive T cells in a sample, the kit comprising:
i. a loadable detection molecule as described herein or a composition as described herein,
ii. at least one antigenic peptide, and
iii. optionally, instructions for use.

An embodiment of the present invention relates to the kit as described herein, wherein the detection molecules have been pre-loaded with antigenic peptides. In this embodiment, the kit does not comprise additional antigenic peptides. Another embodiment of the present invention relates to the kit as described herein, wherein the loadable detection molecules are immobilized on a solid substrate, such as a microarray.

The promiscuous nature of T-cell receptors (TCRs) is fundamental for our ability to recognize a large range of pathogens; however, this feature makes it challenging to understand and control T-cell recognition. Existing technologies provide limited information about the key requirements for T-cell recognition and the ability of TCRs to cross-recognize structurally related elements. The detection molecules described herein serves as a 'one-pot' strategy to establish the patterns that govern TCR recognition of pMHC. Detection molecules as described herein may be used to determine the affinity-based hierarchy of TCR interactions with MHC loaded with peptide variants, and apply this knowledge to understand the recognition motif, herein termed the TCR fingerprint. The loadable detection molecules are a flexible solution perfectly suited for the fast preparation of large libraries of antigenic peptides necessary for high quality TCR fingerprinting. Determination of TCR fingerprints is valuable strategy for understanding T-cell interactions and assessing potential cross-recognition prior to selection of TCRs for clinical development. Thus, an aspect of the present invention relates to a method for determining the interaction between a T cell receptor (TCR) or antigenic peptide responsive T cell and a library of antigenic peptides, the method comprising the following steps:
i. providing loadable detection molecules as described herein,
ii. providing a library of antigenic peptides,
iii. contacting the loadable detection molecules with the library of antigenic peptides to provide a library of loaded detection molecules comprising peptide-MHC (pMHC) class I molecules,
iv. contacting the T cell receptor (TCR) or antigenic peptide responsive T cell with the library of loaded detection molecules, and
v. detecting binding of the T cell receptor (TCR) or antigenic peptide responsive T cell with the library of loaded detection molecules.

This method may also be referred to as "a method for obtaining a TCR fingerprint". Thus, an embodiment of the present invention relates to the method for obtaining a TCR fingerprint, wherein the library of antigenic peptides comprises at least 25 different antigenic peptides, such as at least 50, such as at least 100, such as at least 500, such as at least 1.000, such as at least 10.000 different antigenic peptides. Another embodiment of the present invention relates to the method for obtaining a TCR fingerprint, wherein the library of antigenic peptides comprises at least 100.000 different antigenic peptides, such as at least 1.000.000 different antigenic peptides.

Given the flexible platform for fast generation of a variety of antigenic peptide libraries provided by the methods and loadable detection molecules described herein, it is possible to establish detailed TCR fingerprints against large complex and/or diverse antigenic peptide libraries in a simple on demand manner. Thus, an embodiment of the present invention relates to the method for obtaining a TCR fingerprint, wherein the library of antigenic peptides comprises the human peptidome. Another embodiment of the present invention relates to the method for obtaining a TCR fingerprint, wherein the library of antigenic peptides is generated from a target antigenic peptide and single position variations of said target antigenic peptide. A further embodiment of the present invention relates to the method for obtaining a TCR fingerprint, wherein the library of antigenic peptides is selected from naturally occurring peptides.

Antigenic peptides suitable for the method for obtaining a TCR fingerprint include, but is not limited to, antigenic peptides known or suspected to be pathogenic. Thus, an embodiment of the present invention relates to the method for obtaining a TCR fingerprint, wherein the target antigenic peptide is selected from a cancer-associated epitope, a virus epitopes and a self-epitope. Another embodiment of the present invention relates to the method for obtaining a TCR fingerprint, wherein the cancer-associated epitope is a virus epitope associated with a virus-induced cancer. A further embodiment of the present invention relates to the method for obtaining a TCR fingerprint, wherein the virus epitope is from a virus selected from the group consisting of human papillomavirus (HPV), Merkel cell polyomavirus (MCV), cytomegalovirus (CMV), Epstein-Barr virus (EBV), human T-lymphotropic virus (HTLV), hepatitis B virus (HBV), hepatitis C virus (HCV) and influenza virus.

Another aspect of the present invention relates to use of a loadable detection molecule as described herein for determining the interaction between a T cell receptor (TCR) or antigenic peptide responsive T cell and a library of antigenic peptides.

It should be noted that embodiments and features described in the context of one of the aspects of the present invention also apply to the other aspects of the invention.

The invention will now be described in further details in the following non-limiting examples.

### Examples

### Example 1: Production of Cys-mutant MHC molecules and assembly of detection molecules for antigen specific T cell detection

Here is described how to produce wt (wild type) MHC class I molecules and Cys-mutant MHC class I molecules and the process to make detection molecules according to the present invention. In this example, both wt MHC as well as disulfide stabilized Cys-mutant MHC are prepared and used for assembly of detection molecules.

In this example detection molecules are prepared using fluorophore labelled streptavidin molecules that are commercially available, from for example BD Biosciences, with a range of fluorophores, such as Streptavidin - Phycoerythrin (PE), Streptavidin - Allophycocyanin (APC) etc.

Both wt MHC and Cys-mutant MHC are produced by classical *E.coli* expression, here exemplified using the heavy chain of wt HLA-A (SEQ ID NO:1) and the heavy chain of Cys-mutant HLA-A (SEQ ID NO:2), respectively. Briefly, both wt MHC and MHC Cys-mutant protein and light chain beta-2 microglobulin (B2M) protein are produced in *E.coli* using pET series plasmid. Inclusion bodies containing the *E.coli* produced proteins are harvested by sonication, lysing the *E.coli* cells in lysis buffer (Tris Cl 50 mM, pH 8.0, 1 mM EDTA, 25% sucrose). Soluble fraction of denatured protein is harvested from inclusion bodies by washing in detergent buffer (Tris-Cl 20 mM, pH 7.5, 200 mM NaCl, 2 mM EDTA, NP40 1%, Deoxycholic acid 1%), and wash buffer (0.5% Triton X-100, 1 mM EDTA), followed by 48 hour incubation at 4°C in re-solubilization buffer (HEPES 50 mM, pH 6.5, 8 M urea, 0.1 mM β-mercaptoethanol) and stored in -80°C until used for MHC class I monomer production.

Cys-mutant MHC monomers are made by *in vitro* folding (folding buffer: 100 mM Tris-Cl, pH 8.0, 400 mM L-arginine, 2 mM EDTA, and protease inhibitor cocktail) of Cys-mutant MHC protein and B2M in the presence of a helper molecule, such as *e.g.* a dipeptide/tripeptide/small molecule. wt MHC are made like Cys-mutant MHC monomers except that no helper molecules are included. Instead the wt MHC molecule is refolded in the presence of the UV-labile peptide KILGFVF-X-V (SEQ ID NO:17), where X refers to a UV sensitive amino acid, 3-amino-2-(2-nitrophenyl) propionic acid, which upon exposure to UV light breaks the full length peptide. The reduced binding affinity of the resulting cleaved peptide fragments to the MHC molecules enables binding of incoming exchange antigenic peptide.

MHC monomers are purified by conventional size exclusion chromatography. Alternatively, other standard protein purification methods could be used. Purification of Cys-mutant MHC monomers by size exclusion chromatography can be performed using buffers with or without MHC monomer stabilizing helper molecules.

pMHC are biotinylated by both standard chemical and enzymatic protocols. In these examples, pMHC are enzymatically biotinylated, according to protocol from Avidity Inc., by including a biotinylation consensus peptide sequence in the MHC heavy chain allowing site-specific biotinylation using BirA enzyme (available from Avidity Inc.) and free biotin during the purification process.

In the examples described herein, antigen-specific MHC detection molecules of wt and Cys-mutant MHC or loadable detection molecules of Cys-mutant MHC are assembled via the streptavidin-biotin interaction. Briefly, biotinylated molecules and fluorophore conjugated streptavidin are combined in aqueous buffer, such as PBS, in relative stoichiometry (100 µg/mL MHC molecules mixed with 18 µg/mL of streptavidin-fluorophore conjugates) according to the examples described below and incubated for 30 minutes on ice. Assembled detection molecules can be stored at 4°C for at least one week and -20°C for longer storage. The detection molecules are functionally stable for at least six month at this temperature.

Antigen specific T cells are detected from human PBMCs or TILs using these detection molecules, as detailed in below examples. 2-5x10⁶ cells are incubated with detection molecules and incubated for 15 minutes at 37°C, followed by staining for cell surface markers antibodies for 30 minutes at 4 °C. Cells are then washed twice with FACS buffer (PBS with 2% FCS) and presence of antigen specific CD8+ T cells are analysed by acquiring stained cells on flow cytometer.

### Example 2: Detection molecules prepared with Cys-mutant MHC molecules provide superior and improved antigen specific T-cell detection

This example details out the process of antigen specific CD8+ T cell detection from healthy donor PBMCs using detection molecules, wherein detection molecules are prepared with Cys-mutant MHC molecules. Detection molecules prepared with Cys-mutant MHC molecules provide significantly brighter staining, which is calculated as staining index, as compared to detection molecules prepared from wt MHC monomers for all the detected antigen specific T cell specificities. This was consistently found also with different color fluorophores. In figure 1, flow cytometry data is used to compare detection molecules prepared with wt and Cys-mutant MHC monomers, respectively, for antigen specific T-cell detection in healthy donor PBMC samples.

Healthy donor PBMCs samples are subjected to detection molecules to detect CMV pp65 NLVPMVATV (SEQ ID NO:18, CMV pp65 NLV) antigen-specific CD8+ T cells. HLA A*02:01 (A2) specific CMV pp65 NLVPMVATV detection molecules are prepared using wt and Cys-mutant A2 molecules and compared for T cell detection efficiency. wt and Cys-mutant A2 molecules are produced and purified as described in Example 1. To make CMV pp65 NLVPMVATV antigen specific detection molecules, 2 µM wt A2 molecules are incubated with 100 µM CMV pp65 NLVPMVATV peptide under a UV lamp (365 nm) for one hour to facilitate UV-light mediated peptide exchange to make A2 CMV pp65 NLVPMVATV monomers (as wt A2 molecules carries UV-labile peptide added during folding and required stability of these molecules, thus requires UV-mediated exchange process).

For Cys-mutant MHC molecules, 100 µM CMV pp65 NLVPMVATV is directly incubated with 2 µM Cys-mutant A2 molecules for one hour at 4°C. Cys-mutant MHC molecules does not require any exchange process, as these molecules are stable in peptide-free conformation after folding, which allows direct loading of the peptide of choice without the need of any exchange process. Thus, Cys-mutant MHC molecules are ideal for direct, easy, and fast loading of antigenic peptides for T cell detection.

A2 CMV pp65 NLVPMVATV monomers of wt and Cys-mutant are then converted to MHC detection molecules by incubating 2 µM A2 CMV pp65 NLVPMVATV with 18 µg/mL fluorophore conjugated streptavidin for 30 minutes at 4°C. PBMCs from healthy donors are stained using these wt and Cys-mutant A2 CMV pp65 NLVPMVATV specific detection molecules. Representative dot plots, showing A2 CMV pp65 NLVPMVATV specific CD8+ T cells, are shown in figure 1A.

Brighter staining detected for A2 CMV pp65 NLVPMVATV-specific T cells by Cys-mutant MHC detection molecules is validated by calculated the staining index, *i.e.* better separation of detection molecule positive (antigen-specific) T cells from the background signal. A nearly three-fold higher staining index is observed for the Cys-mutant detection molecules over wt detection molecules (Figure 1B). Superiority of detection molecules prepared from Cys-mutant MHC molecules is detected in two additional healthy donor samples with a different fluorophore tag (Figure 2A). Irrespective of which fluorophore tagged detection molecule was used, Cys-mutant A2 CMV pp65 NLVPMVATV-specific detection molecules performed superior to wt MHC detection molecules in both tested healthy donor samples, determined using staining index calculation (Figure 2B).

Superiority in staining index of Cys-mutant T-cell detection molecules is found across several antigen specificities, as shown by detecting antigen specific T cells for two more virus specific antigens in healthy donor PBMCs. A2 YVLDHLIVV (SEQ ID NO:19, EBV BRLF1 YVL), and A2 VLEETSVML (SEQ ID NO:20, CMV IE1 VLE) antigen specific detection molecules assembled using wt A2 or Cys-mutant A2, as described previously in this example. Healthy donor PBMCs are incubated with the detection molecules and staining index is compared for wt and Cys-mutant detection molecules. Cys-mutant MHC detection molecules provides much superior staining index (4-5 folds higher) for both type of antigen specific CD8 T cells (A2 YVLDHLIVV and A2 VLEETSVML) (Figure 3A, B).

**Conclusion:** This experiment demonstrates the feasibility of detecting antigen specific T cells using detection molecules prepared with Cys-mutant MHC molecules (Figure 1). T cell detection efficiency of Cys-mutant MHC detection molecules is found comparable to the wt detection molecules.

As T-cell detection efficiencies are comparable between Cys-mutant and wt MHC detection molecules, an advantage with Cys-mutant MHC molecules is the faster loading of antigenic peptide. Cys-mutant MHC molecules are folded empty and does not require a full length peptide to be stable. Thus, Cys-mutant MHC molecules are peptide receptive and can be loaded with any antigenic peptide without the use of a peptide exchange technology or individual folding and purification of each pMHC specificity, as required for wt MHC molecules.

In addition another advantage of Cys-mutant detection molecules comes from the better T-cell detection efficiency as demonstrated via staining index (Figure 1B, 2B, and 3B). A three- to eight-fold higher staining index is shown by Cys-mutant A2 detection molecules over the wt A2 detection molecules across three different antigens (A2-NLVPMVATV, A2 YVLDHLIVV and A2 VLEETSVML), in three different donor samples (BC60, 89, 90), and with two different colors (PE, and APC). These data provide an advancement in antigen specific T cell detection by MHC detection molecules using Cys-mutant MHC detection molecules, providing an improved distinction in T-cell detection over existing approaches.

Figure 3A demonstrates the strength of this advancement in T-cell detection using Cys-mutant MHC molecules. Low frequency A2 YVLDHLIVV (EBV BRLF1) specific T-cells, detected in healthy donor PBMCs, are clearly distinguished and well separated from the detection molecule negative population when Cys-mutant detection molecules were used due to brighter staining (*i.e.* better staining index). In contrast, wt A2 detection molecules used to detect the same antigen specific T cells, provided poor separation of detection molecule positive cells, which is so undistinguishable that one can almost not detect this population. Thus, a brighter separation (improved staining index) is important to identify low frequency and low affinity antigen specific T cells, which is being facilitated by the use of Cys-mutant MHC molecules thus providing a clear advancement from the existing approaches.

### Example 3: Combicoding detection molecules made from Cys-mutant MHC molecules offers a significant improvement for detection of antigen specific T-cell.

In this example is demonstrated an advantage of applying a combinatorial setup of detection molecules, termed combicoding, for detection of multiple T cell specificities in a sample. Combicoding is particular advantageous for Cys-mutant MHC detection molecules as compared to conventional wt MHC detection molecules. It is demonstrated that in a combinatorial setup, where one fluorophore tag is used to detect several antigens in combination with a second fluorophore tag which is specific to only one antigen, thereby forming a unique two color combination for each antigen specificity, a better staining index ratio for Cys-mutant/wt detection molecules is observed. When screening large peptide libraries by combicoding a clear separation of detection molecule positive T-cells facilitates better identification of antigen specific T cells from large pools of cells in particular when they are rare and with low affinity to a given antigenic peptide MHC combination. The combicoding technique has previously been described by Hadrup *et al.* (2009) and in WO 2010/060439 A1.

In a combicoding example, wt and Cys-mutant A2 detection molecules are compared for T cell detection efficiency across three different antigens; A2-FLU MP GILGFVFTL (SEQ ID NO: 21, FLU MP GIL), A2-EBV BMF1 GLCTLVAML (SEQ ID NO: 22, EBV BMF1 GLC), and A2 EBV LMP2 FLYALALLL (SEQ ID NO: 23, EBV LMP2 FLY). All three antigen-specific detection molecules are prepared similar to the process described in example 2. Briefly, 200 µM of each antigenic peptide is incubated with 2 µM wt or Cys-mutant A2 molecules for one hour either under UV lamp (for wt A2 exchange reaction) or directly at 4°C (Cys-mutant A2). After one hour, each of the antigen specific monomers are split into two reactions, and each is incubated with a different color fluorophore conjugated streptavidin for 30 minutes. For all three antigen specificities APC is used as first color and as second color BUV395 (for A2-FLU MP GILGFVFTL), BUV737 (for A2-EBV BMF1 GLCTLVAML), and PE (A2 EBV LMP2 FLYALALLL). Thus, this dual color approach gives a unique color combination to each antigen specific detection molecule; APC-BUV395 for A2-FLU MP GILGFVFTL, APC-BUV737 for A2-EBV BMF1 GLCTLVAML, and APC-PE for A2 EBV LMP2 FLYALALLL.

Prior to staining the cells, dual-color combicoding detection molecules for each antigen specificity across all three are pooled in equal amount (1 µL/color/specificity). Pooled detection molecules are then incubated with 2-5x10⁶ cells for 15 minutes at 37°C, and 30 minutes at 4°C with cell surface marker antibodies. Cells are then washed and acquired on flow cytometry for CD8+ antigen-specific T cells. Figure 4A; dot plots demonstrate the comparative antigen specific T cell detection efficiency of wt and Cys-mutant A2 detection molecules. Staining indexes are calculated for each detected antigen specific T cell plot for both the colors in a given combination (Figure 4B). Dot plots and staining index bar plots (fold change Cys-mutant/wt A2 detection molecules) demonstrate the improved detection using Cys-mutant detection molecules as compared to wt detection molecules.

Cys-mutant MHC detection molecules are also advantageous for identifying cancer associated antigen specific T cells from melanoma patient samples in a combicoding setup (Figure 5). A library of 12 melanoma associated cancer antigens (HLA A*02:01 restricted) is analysed to identify CD8+ T cells in melanoma patient TILs (tumor infiltrating lymphocytes). A panel of combicoding detection molecules covering the 12 antigens is generated, wherein each pMHC combination is tagged with two color detection molecules as described above in this example. Prior to staining, 1 µl of each pMHC detection molecules of two color combination is pooled in a single tube and mixed with 2x10⁶ melanoma patient TILs and incubated for 15 minutes at 37°C. Cells are then acquired on flow cytometer after incubating with cell surface antibodies for 30 minutes at 4°C. Figure 5 dot plots demonstrate the CD8+ T cells identified specific for two of the cancer associated antigens (ITDQVPFSV (SEQ ID NO:24, gp100 ITD) and AMLGTHTMEV (SEQ ID NO:25, gp100 AML)) and provides a comparison between Cys-mutant and wt A2 detection molecules. Cys-mutant detection molecules consistently provides superior staining index for all the identified antigen specific T cell specificities, further validating an advantage of Cys-mutant MHC detection molecules in combicoding.

**Conclusion:** This example demonstrates the usefulness of detection molecules prepared with Cys-mutant MHC molecules for identifying and detecting antigen specific CD8 T cells by combicoding. A strength of the Cys-mutant MHC detection molecules lies in the better staining index, thus creating better separation from the negative population of T cells. This feature is very important for the combicoding because a single fluorophore tag can be represented by multiple antigen specific detection molecules, thus in a final flow cytometer readout a single colored detection molecule positive T cell population can represent more than one antigen specific T cell. These cells can only be segregated by identifying the second color of the detection molecule pair. Hence, a better separation and higher staining index gives a definitive advantage as shown by Cys-mutant detection molecules.

An advantage offered by Cys-mutant MHC detection molecules is demonstrated by the identification and detection of cancer associated antigens in melanoma patient samples (Figure 5). A high throughput approach, such as MHC detection molecule based combicoding, is required to screen large peptide libraries to identify cancer associated antigen specific T cells in patient samples for immunotherapeutic development. Cys-mutant MHC detection molecules are better suited for such an approach as they can be loaded much faster when large peptide libraries are required for screening and they provide improved staining index also for cancer-associated antigens. An improved staining index for cancer-associated antigens also has a great advantage due to the wide range of possible pMHC and T cell receptor interactions.

### Example 4: Loadable detection molecules prepared with Cys-mutant MHC molecules provide a fast way to generate antigen-specific detection molecules for antigen specific T cell detection by combicoding

This example demonstrates a fast way to make detection molecules using Cys-mutant MHC for screening large antigen libraries to identify antigen specific CD8 T cells. Since Cys-mutant MHC molecules are highly stable and peptide receptive, unlike wt MHC molecules, these empty monomers can rapidly be converted to ready detection molecules (Figure 6A). This process comprises generating loadable detection molecules, which can be stored at -20 °C. These loadable detection molecules can be loaded with the choice of antigenic peptide by a single step incubation. The present example demonstrates that this process can be achieved in as little as one minute and that such antigenic peptide specific detection molecules are functionally active in T cell detection. Cys-mutant A2 monomers are produced as described in example 1. Cys-mutant MHC monomers (100 µg/mL) are then incubated with fluorophore tagged streptavidin (18 µg/mL) for 30 minutes at 4°C to generate loadable detection molecules. Loadable detection molecules are stored in -20°C until used for making antigen specific detection molecules in storage buffer (0.5 % BSA, 5% Glycerol).

To test the peptide loading efficiency and for functional validation of loadable A2 detection molecules, loadable MHC detection molecules (APC labelled) are converted to pMHC detection molecules by incubating varying concentration of peptide (0.1, 1, 10, 100 µM) for different incubation time (1, 5, 15, 30, and 60 minutes) at 4 °C. In this example (Figure 6B), A2 GILGFVFTL (FLU MP) antigen is used to detect the antigen specific T cells. A2 GILGFVFTL Cys-mutant detection molecules from each peptide loading condition are incubated with healthy donor PBMCs (2x10⁶ cells/staining). Figure 6B demonstrates A2 GILGFVFTL specific T-cells detected in the donor sample by each peptide loading condition. The data shows that peptide loading for one minute is sufficient to make antigen specific detection molecule that can detect its restricted T cells.

In this example, the application of loadable detection molecule in combicoding to identify neoantigen reactive CD8 T cells in melanoma patient samples is further verified. Neoantigens derives from cancer specific mutations and thus could potentially find use in various immunotherapeutic approaches. Identification of neoantigen specific T cells requires screening large peptide libraries specific to individual patients. Therefore, to screen such large peptide libraries a high throughput system would require fast preparation of detection molecules. In this example, it is demonstrated that loadable detection molecules made from Cys-mutant MHC molecules are ideal for such approaches. This is exemplified by generating a melanoma patient specific neoantigen library of 43 peptide antigens (HLA-A2 restricted) in a combicoding setup. Loadable detection molecules (in two color combination) are incubated with 100 µM neoantigen and incubated for 15 minutes and pooled immediately and incubated with melanoma TILs. Cells are stained as described previously and acquired by flow cytometer. By combicoding with loadable detection molecules, CD8 T cells specific for three neoantigens (RLMVAVEEA (SEQ ID NO:26, VANGL1 RLM), RLMVAVEEAFI (SEQ ID NO:27, VANGL1(2) RLM) and LLLFLTIFI (SEQ ID NO:28, DYSF LLL)) are identified (Figure 7).

**Conclusion:** An advantage of using Cys-mutant MHC detection molecules in combicoding to identify neoantigen specific T cells is demonstrated in this example. Other existing approaches in the prior art require an exchange method to load any peptide of interest prior to making detection molecules, thus making the process inefficient and very time consuming especially when large patient-specific peptide libraries needs to be analysed. Loadable detection molecules that can be converted in a single step into antigen specific detection molecules provide an advantageous fast solution to the problematic approach of existing solutions. In this example is identified neoantigen specific T-cells using rapidly converted loadable A2 detection molecules into antigen specific detection molecules in just 15 minutes, which is a great improvement over any of the existing methods of detection-molecule based T cell detection.

### Example 5: Detection molecules prepared with Cys-mutant MHC molecules are highly stable as compare to detection molecules prepared with wild-type MHC

This example shows that Cys-mutant MHC detection molecules are highly stable as compared to wt MHC detection molecules. Higher stability of antigen specific MHC detection molecules improves T cell detection especially for low affinity antigens using combicoding and barcoding methods for T-cell detection.

In this example the improved stability of Cys mutant antigen specific detection molecules is demonstrated by peptide dissociation measurements and compared with the wt antigen specific detection molecules. Loadable detection molecules (APC tagged) are prepared from Cys-mutant A2 MHC molecules, as described in example 4. 10 µM GILGFVFTL (FLU MP) peptide is incubated for 15 minutes at 4°C with loadable A2 detection molecules to generate A2-GILGFVFTL antigen specific detection molecules. Wt A2-GILGFVFTL antigen specific detection molecules are generated by subjecting wt A2 monomers under the UV lamp (365 nm) for 1 hour in the presence of 200 µM GILGFVFTL peptide, followed by incubating with streptavidin APC for 30 minutes at 4°C. After antigen specific detection molecule preparation, excess peptides are removed for both wt and Cys-mutant detection molecules using molecular weight cut-off filters (commercially available). Resulting A2-GILGFVFTL specific detection molecules are incubated at 4°C, room temperature, and 37°C for 1 hour, 12 hours, 48 hours, and 168 hours to determine the stability of pMHC complexes. Stability of these complexes after each time point and incubation temperature is measured as functional readout to detect A2 GILGFVFTL antigen specific T cells from healthy donor PBMCs. T cell staining is performed as described in previous examples.

A2-GILGFVFTL detection molecules prepared using Cys mutant A2 molecules are found to be distinctively more stable than the wt detection molecules of the same specificity. A clear distinction is observed for detection molecules when incubated at 37°C incubated 12 hours or more, with A2-GILGFVFTL specific T cell detection efficiency decreasing significantly for wt detection molecules. In contrast, Cys-mutant detection molecules incubated at 37°C for up to 48 hours are still capable of detecting the A2 GILGFVFTL specific T cells (Figure 8 A, B).

**Conclusion:** Cys-mutant detection molecules are highly stable, as measured using peptide dissociation rate. Even after incubating at 37°C for 48 hours the Cys-mutant detection molecules are functionally stable and efficiently detect antigen specific T cells. The higher stability of Cys-mutant pMHC complexes gives them a distinct advantage over wt pMHC complexes in high throughput T cell detection platforms such as by combicoding and barcoding of detection molecules since the low affinity pMHC-TCR (T cell receptor) interactions are more likely to be detected using more stable pMHC complexes.

### Example 6: Detection molecules prepared using Cys-mutant MHC molecules provide significant advantages in barcoding based high throughput T cell detection method

This example describes detection of antigen specific T cells using barcoded Cys-mutant MHC molecules and wt MHC molecules detection molecules. The barcoding method utilizes unique short nucleic acid sequences as barcodes attached to each type of pMHC detection molecule. This method allows screening of thousands of antigenic peptides in a single pot assay using nucleic acid barcoded T-cell detection molecules. This technique has previously been described by Bentzen *et al.* (2016) and in WO 2015/188839 A2.

In this example, T cells specific for cancer associated antigens in healthy donor (Figure 9) and melanoma patient samples are detected (Figure 10). DNA barcoded detection molecules (wt and Cys-mutant) are prepared for 167 A2 restricted cancer antigens and eight A2 restricted virus derived antigens.

DNA barcodes are created from oligonucleotides containing distinct 25mer nucleotide sequences. In addition to the distinct 25mer region, each oligo contains primer regions compatible with IonTorrent sequencing as well as a 6xN random nucleotide unique molecular identifier (UMI) region. Oligonucleotides modified with a 5' biotin tag (A barcodes) are joined to unmodified, partially complementary oligonucleotides (B barcodes) to generate 175 unique double-stranded Ax-By DNA barcodes (5 representative A barcode (SEQ ID NOs:32-36) and B barcode (SEQ ID NOs:37-41) sequences and the antigenic peptides they encode are listed in Table 1, "N" denotes a random nucleotide). Combinations of A and B oligos (one of each) are mixed with 5x Sequenase Reaction Buffer mix (PN 70702, Affymetrix) to a final concentrations of 26 µM (Oligo A) and 52 µM (Oligo B), respectively; heated to 65 °C for 2 min; and allowed to anneal by cooling slowly to <35 °C over 15-30 min. The annealed oligo As and Bs are elongated to create double-stranded AxBy DNA barcodes by adding Sequenase polymerase (70775Y, Affymetrix), 20 µM DTT and 800 µM or 72 µM dNTPs, followed by incubation for 5-10 min at RT. Elongated AxBy barcodes are diluted in nuclease-free water + 0.1% Tween to 2.17 µM (with respect to the A oligo) and stored at -20 °C. 5' biotinylated AxBy DNA barcodes are attached to PE- and streptavidin-conjugated dextran in a 2:1 stoichiometry. DNA barcodes are attached by mixing with dextran-conjugate, followed by incubation, 30 min at 4 °C.

Individual pMHC combinations are prepared for each of the 175 antigens by loading these antigens to A2 molecules. For wt A2 molecules, antigens are loaded using UV-mediated peptide exchange process, wherein 100 µg/mL wt A2 monomers are incubated with 200 µM peptide for one hour under UV light (365 nm). Cys-mutant A2 molecules are loaded with antigens by incubating 100 µM peptide antigens for just 15 minutes without the requirement of any exchange process.

| **Table 1** | | | |
|---|---|---|---|
| **Peptide encoded** | **Barcode name** | **A-barcode sequence 5'-3'** | **B-barcode sequence 5'-3'** |
| VLYRYGSFSV | A22-B63 | (SEQ ID NO:32) Biotin-C6- GAAGTTCCAGCCAGCGTCAC AGTTTNNNNNNTAATACGCC TGAGGTGTTGGGTTGCGGTC AGCATCATTTCC | (SEQ ID NO:37) GTTATCGGCTCGTTCACACTC GANNNNNNCCGGTTTTATAC CCTCGTTCCCCGAGGAAATG ATGCTGACC |
| RLMKQDFSV | A22-B64 | (SEQ ID NO:33) Biotin-C6- GAAGTTCCAGCCAGCGTCAC AGTTTNNNNNNTAATACGCC TGAGGTGTTGGGTTGCGGTC AGCATCATTTCC | (SEQ ID NO:38) GTTATCGGCTCGTTCACACTC GANNNNNNCAGAACTACAGG CTGGCATGGATGCGGAAATG ATGCTGACC |
| RLPRIFCSC | A22-B65 | (SEQ ID NO:34) Biotin-C6- GAAGTTCCAGCCAGCGTCAC AGTTTNNNNNNTAATACGCC TGAGGTGTTGGGTTGCGGTC AGCATCATTTCC | (SEQ ID NO:39) GTTATCGGCTCGTTCACACTC GANNNNNNATTCTGATGGGT AGAAACCGTTCCCGGAAATG ATGCTGACC |
| ALLEIASCL | A22-B71 | (SEQ ID NO:35) Biotin-C6- GAAGTTCCAGCCAGCGTCAC AGTTTNNNNNNTAATACGCC TGAGGTGTTGGGTTGCGGTC AGCATCATTTCC | (SEQ ID NO:40) GTTATCGGCTCGTTCACACTC GANNNNNNTTTCCTTGTTCG GATCGGTCGAGAAGGAAATG ATGCTGACC |
| KASEKIFYV | A22-B75 | (SEQ ID NO:36) Biotin-C6- GAAGTTCCAGCCAGCGTCAC AGTTTNNNNNNTAATACGCC TGAGGTGTTGGGTTGCGGTC AGCATCATTTCC | (SEQ ID NO:41) GTTATCGGCTCGTTCACACTC GANNNNNNCATATGGATTTG TTGCATCCTGATGGGAAATGA TGCTGACC |

| **Forward primer** | | **Sequence 5'-3'** | |
|---|---|---|---|
| A-Key 2OS-F1-233 | | CCATCTCATCCCTGCGTGTCTCCGACTCAGTGACAGGTTGGAA GTTCCAGCCAGCGTC (SEQ ID NO:42) | |

| **Reverse primer** | | **Sequence 5'-3'** | |
|---|---|---|---|
| P1R2 | | GTTATCGGCTCGTTCACACTCGA (SEQ ID NO:43) | |

Assembled pMHC specificities are then incubated for 30 minutes with streptavidin conjugated dextramers (tagged with DNA barcodes) at 4°C to get DNA barcoded antigen specific detection molecules. All 175 different pMHC-specific barcoded T cell detection molecules are then pooled in a single tube (1.5 µL from each specificity) and incubated with healthy donor PBMCs or melanoma patient TIL samples (2-5x10⁶ cells) for 15 minutes at 37°C. Cells are stained with cell surface antibodies as described previously and acquired on cell-sorter to separate antigen specific T-cell positive for detection molecules. From these sorted cells, DNA barcodes are amplified using IonTorrent compatible forward A-Key 2OS-F1-xxx and reverse PCR primer P1R2 (a single forward primer A-Key 2OS-F1-233 (SEQ ID NO:42) and the reverse primer P1R2 (SEQ ID NO:43) are exemplified in Table 1). PCR amplified DNA barcodes are purified and sequenced by IonTorrent sequencing to identify antigen specific T-cell associated with respective DNA barcodes essentially as described in Bentzen *et al.* (2016).

Barcoded detection molecules assembled with Cys-mutant A2 molecules overall detect higher numbers of antigen-specific CD8 T cells compared to detection molecules assembled using wt A2 molecules. In healthy donor both wt A2 and Cys-mutant A2 pMHC detected similar number of T-cells specific to virus derived antigens, but Cys-mutant detection molecules identify several additional cancer associated antigen specific T-cells in this samples (Figure 9), demonstrating the sensitivity of Cys-mutant MHC molecules. Notably, in both the melanoma patient samples wt A2 detection molecules did not identify any antigen specific T cells, whereas Cys-mutant MHC detection molecules identified one cancer antigen specific T-cells in melanoma patient one and two in the second melanoma patient sample (VLYRYGSFSV (SEQ ID NO:29, MEL VLY) and KASEKIFYV (SEQ ID NO:30, MEL KAS)) (Figure 10).

**Conclusions:** This example (Figure 9 and 10) demonstrates that barcoded detection molecules with Cys-mutant MHC molecules provides an attractive platform for high throughput T-cell detection. T-cells specific to cancer antigens are very difficult to detect, possibly due to low frequency or low affinity interaction between pMHC or TCR. Cys-mutant MHC molecules outperform wt MHC detection molecules for high throughput T-cell detection, as only Cys-mutant T-cell detection molecules are sensitive enough to detect rare cancer-associated antigen specific T cells in melanoma patient samples.

### Example 7: DNA barcoded detection molecules prepared using Cys-mutant MHC molecules to determine TCR fingerprint

This example describes the use of Cys-mutant MHC molecules to determine T-cell receptor (TCR) fingerprint. TCR fingerprints provide the details of TCR-pMHC interaction and importance of each residue in T-cell recognition. The example demonstrates that Cys-mutant MHC molecules identifies TCR fingerprint comparable to TCR fingerprint identified using wt MHC molecules.

To determine TCR fingerprints, barcoding of detection molecules is used to characterize 192 peptides arriving from Merkel Cell Polyomavirus-derived epitope, A2-KLLEIAPNY (SEQ ID NO:31, MCV KLL) by the sequential replacement of each amino acid position by the 20 naturally occurring amino acids. Each of these peptide variants is loaded either onto Cys-mutant A2 empty monomers or replaced for a UV-cleavable ligand in wt A2. Then, MHC multimers are generated and tagged using 192 unique DNA barcodes generated as described in example 6. A mixture of all pMHC multimer combinations is used to stain the T-cell clones (specific to A2-KLLEIAPNY), allowing for a measure of the affinity-based TCR-pMHC interaction and consequently providing the TCR's recognition preferences. The TCR fingerprint was determined based on the sequencing of the MHC multimer-associated DNA barcodes, and the results are given as logFc to the input pMHC library. LogFc is converted to Shannon logos to better illustrate the aminoacid requirements for TCR pMHC interactions (Figure 11). Based on TCR fingerprint, both Cys-mutant and wt A2 showed comparable TCR fingerprint pattern for both the tested TCR clones specific to A2-KLLEIAPNY.

**Conclusion:** This example describes the application of Cys-mutant MHC molecules for TCR fingerprint analysis. It demonstrates that Cys-mutant MHC molecules accurately reproduce TCR fingerprints and are better suited for such analysis as they can be loaded with desired antigenic peptide quickly in a single step. This is a clear advantage in such high throughput barcoding methods where it is required to screen large peptide libraries.

### References

- Hadrup et al. (2009), Nat. Methods. 6, 520-526
- WO 2010/060439 A1
- Bentzen et al. (2016), Nat. Biotechnol. 34, 1037-1045
- WO 2015/188839 A2
- Altman et al. (1996), Science, 274, 94-96

## Claims

1. A method for detection of one or more antigenic peptide responsive T cells in a sample, the method comprising the following steps:
i. providing loadable detection molecules comprising at least one peptide-free MHC class I molecule and at least one detectable label,
ii. providing at least one antigenic peptide,
iii. contacting the loadable detection molecules with the at least one antigenic peptide to provide loaded detection molecules comprising at least one peptide-MHC (pMHC) class I molecule,
iv. contacting the loaded detection molecules with the sample, and
v. detecting binding of the loaded detection molecules to the one or more antigenic peptide responsive T cells,
wherein each of the at least one antigenic peptide is represented by:
- at least two different detectable labels, or
- at least one detectable label which is a nucleic acid label,
wherein the peptide-free MHC class I molecule comprises a heavy chain comprising an alpha-1 domain and an alpha-2 domain connected by a disulfide bridge, said heavy chain comprising an amino acid sequence selected from:
(a) SEQ ID NO: 12, or
(b) an amino acid sequence having at least 80%, 85%, 90%, or 95% sequence identity to the sequence in (a),
wherein a mutant cysteine residue is positioned in the alpha-1 domain at amino acid residue 84 or 85 and a mutant cysteine residue is positioned in the alpha-2 domain at amino acid residue 139.

2. The method according to claim 1, wherein the peptide-free MHC class I molecule is attached to a connector molecule via non-covalent interactions between the connector molecule and an affinity tag on the peptide-free MHC class I molecule.

3. The method according to any one of the preceding claims, wherein the loadable detection molecule comprises at least two peptide-free MHC class I molecules, such as at least three peptide-free MHC class I molecules, preferably four peptide-free MHC class I molecules.

4. The method according to any one of claims 2-3, wherein the at least one detectable label is attached to the connector molecule.

5. The method according to any one of the preceding claims, wherein the at least one antigenic peptide is represented by at least two different detectable labels, and binding of the loaded detection molecules to the one or more antigenic peptide responsive T cells is detected by detecting the presence of the at least two different detectable labels bound to an antigen responsive T cell through the loaded detection molecules.

6. The method according to claim 5, wherein the at least two different detectable labels are selected from the group consisting of fluorescent markers, radioisotopes, magnetic markers and metal labels.

7. The method according to any one of claims 1-4, wherein the at least one detectable label is a nucleic acid label comprising
i. a 5 'first primer region (forward), a barcode region and a 3' second primer region (reverse), and
ii. a unique molecular identifier (UMI) region of random nucleotide bases
wherein the barcode region is a unique barcode serving as an identification tag for the detection molecule.

8. A loadable detection molecule comprising at least one peptide-free MHC class I molecule and a nucleic acid label,
wherein the peptide-free MHC class I molecule comprises a heavy chain comprising an alpha-1 domain and an alpha-2 domain connected by a disulfide bridge, said heavy chain comprising an amino acid sequence selected from:
(a) SEQ ID NO: 12, or
(b) an amino acid sequence having at least 80%, 85%, 90%, or 95% sequence identity to the sequence in (a),
wherein a mutant cysteine residue is positioned in the alpha-1 domain at amino acid residue 84 or 85 and a mutant cysteine residue is positioned in the alpha-2 domain at amino acid residue 139.

9. The loadable detection molecule according to claim 8, wherein the nucleic acid label is as defined in claim 7.

10. The loadable detection molecule according to any one of claims 8 or 9, wherein the loadable detection molecule comprises four peptide-free MHC class I molecules attached to streptavidin via non-covalent interactions between streptavidin and a biotin tag on each peptide-free MHC class I molecule.

11. A composition for detection of one or more antigenic peptide responsive T cells in a sample, the composition comprising:
(a) at least two loadable detection molecules according to any one of claims 8-10, or
(b) at least three loadable detection molecules each comprising at least one peptide-free MHC class I molecule and at least one detectable label, wherein each loadable detection molecule comprises a different detectable label,
wherein the peptide-free MHC class I molecule comprises a heavy chain comprising an alpha-1 domain and an alpha-2 domain connected by a disulfide bridge, said heavy chain comprising an amino acid sequence selected from:
(a) SEQ ID NO: 12, or
(b) an amino acid sequence having at least 80%, 85%, 90%, or 95% sequence identity to the sequence in (a),
wherein a mutant cysteine residue is positioned in the alpha-1 domain at amino acid residue 84 or 85 and a mutant cysteine residue is positioned in the alpha-2 domain at amino acid residue 139.

12. Use of a loadable detection molecule according to any one of claims 8-10 or a composition according to claim 11 for detection of one or more antigenic peptide responsive T cells in a sample.

13. A kit for detection of one or more antigenic peptide responsive T cells in a sample, the kit comprising:
i. a loadable detection molecule according to any one of claims 8-10 or a composition according to claim 11,
ii. at least one antigenic peptide, and
iii. optionally, instructions for use.

14. A method for determining the interaction between a T cell receptor (TCR) or antigenic peptide responsive T cell and a library of antigenic peptides, the method comprising the following steps:
i. providing loadable detection molecules according to any one of claims 8-10,
ii. providing a library of antigenic peptides,
iii. contacting the loadable detection molecules with the library of antigenic peptides to provide a library of loaded detection molecules comprising peptide-MHC (pMHC) class I molecules,
iv. contacting the T cell receptor (TCR) or antigenic peptide responsive T cell with the library of loaded detection molecules, and
v. detecting binding of the T cell receptor (TCR) or antigenic peptide responsive T cell with the library of loaded detection molecules.

15. Use of a loadable detection molecule according to any one of claims 8-10 for determining the interaction between a T cell receptor (TCR) or antigenic peptide responsive T cell and a library of antigenic peptides.

## Patentansprüche

1. Verfahren für ein Detektieren einer oder mehrerer auf antigenes Peptid ansprechender T-Zellen in einer Probe, das Verfahren die folgenden Schritte umfassend:
i. Bereitstellen beladbarer Detektionsmoleküle, die mindestens ein peptidfreies MHC-Klasse-I-Molekül und mindestens eine detektierbare Markierung umfassen,
ii. Bereitstellen mindestens eines antigenen Peptids,
iii. Inkontaktbringen der beladbaren Detektionsmoleküle mit dem mindestens einen antigenen Peptid, um beladene Detektionsmoleküle bereitzustellen, die mindestens ein Peptid-MHC-(pMHC)-Klasse-I-Molekül umfassen,
iv. Inkontaktbringen der beladenen Detektionsmoleküle mit der Probe und
v. Detektieren von Bindung der beladenen Detektionsmoleküle an die eine oder die mehreren auf antigenes Peptid ansprechenden T-Zellen,
wobei jedes des mindestens einen antigenen Peptids durch Folgendes dargestellt ist:
- mindestens zwei unterschiedliche detektierbare Markierungen oder
- mindestens eine detektierbare Markierung, die eine Nukleinsäuremarkierung ist,
wobei das peptidfreie MHC-Klasse-I-Molekül eine schwere Kette umfasst, die eine alpha-1-Domäne und eine alpha-2-Domäne umfasst, die durch eine Disulfid-Brücke verbunden sind, wobei die schwere Kette eine Aminosäuresequenz umfasst, die ausgewählt ist aus:
(a) SEQ ID-NR.: 12, oder
(b) einer Aminosäuresequenz mit mindestens 80 %, 85 %, 90 % oder 95 % Sequenzidentität zu der Sequenz in (a),
wobei ein mutierter Cysteinrest in der alpha-1-Domäne an Aminosäurerest 84 oder 85 positioniert ist und ein mutierter Cysteinrest in der alpha-2-Domäne an Aminosäurerest 139 positioniert ist.

2. Verfahren nach Anspruch 1, wobei das peptidfreie MHC-Klasse-I-Molekül über nichtkovalente Wechselwirkungen zwischen dem Konnektormolekül und dem Affinitäts-Tag an dem peptidfreien MHC-Klasse-I-Molekül an dem Konnektormolekül befestigt wird.

3. Verfahren nach einem der vorhergehenden Ansprüche, wobei das beladbare Detektionsmolekül mindestens zwei peptidfreie MHC-Klasse-I-Moleküle, wie z. B. mindestens drei peptidfreie MHC-Klasse-I-Moleküle, vorzugsweise vier peptidfreie MHC-Klasse-I-Moleküle, umfasst.

4. Verfahren nach einem der Ansprüche 2-3, wobei die mindestens eine detektierbare Markierung an das Konnektormolekül gebunden ist.

5. Verfahren nach einem der vorhergehenden Ansprüche, wobei das mindestens eine antigene Peptid durch mindestens zwei unterschiedliche detektierbare Markierungen dargestellt wird und ein Binden der beladenen Detektionsmoleküle an die eine oder die mehreren auf antigenes Peptid ansprechenden T-Zellen durch Detektieren des Vorhandenseins der mindestens zwei unterschiedlichen detektierbaren Markierungen, die durch die beladenen Detektionsmoleküle an eine auf Antigen ansprechende T-Zelle gebunden sind, detektiert wird.

6. Verfahren nach Anspruch 5, wobei die mindestens zwei unterschiedlichen detektierbaren Markierungen aus der Gruppe ausgewählt werden bestehend aus Fluoreszenzmarkern, Radioisotopen, magnetischen Markern und Metallmarkierungen.

7. Verfahren nach einem der Ansprüche 1-4, wobei die mindestens eine detektierbare Markierung eine Nukleinsäuremarkierung ist, umfassend
i. eine 5'-erste Primerregion (vorwärts), eine Barcoderegion und eine 3'-zweite Primerregion (rückwärts) und
ii. eine Region mit eindeutigen molekularen Identifikatoren (UMI) von zufälligen Nukleotidbasen,
wobei die Barcoderegion ein eindeutiger Barcode ist, der als Identifikations-Tag für das Detektionsmolekül dient.

8. Beladbare Detektionsmoleküle, die mindestens ein peptidfreies MHC-Klasse-I-Molekül und mindestens eine Nukleinsäuremarkierung umfassen,
wobei das peptidfreie MHC-Klasse-I-Molekül eine schwere Kette umfasst, die eine alpha-1-Domäne und eine alpha-2-Domäne umfasst, die durch eine Disulfid-Brücke verbunden sind, wobei die schwere Kette eine Aminosäuresequenz umfasst, die ausgewählt ist aus:
(a) SEQ ID-NR.: 12, oder
(b) einer Aminosäuresequenz mit mindestens 80 %, 85 %, 90 % oder 95 % Sequenzidentität zu der Sequenz in (a),
wobei ein mutierter Cysteinrest in der alpha-1-Domäne an Aminosäurerest 84 oder 85 positioniert ist und ein mutierter Cysteinrest in der alpha-2-Domäne an Aminosäurerest 139 positioniert ist.

9. Beladbares Detektionsmolekül nach Anspruch 8, wobei die Nukleinsäuremarkierung wie in Anspruch 7 definiert ist.

10. Beladbares Detektionsmolekül gemäß einem der Ansprüche 8 oder 9, wobei das beladbare Detektionsmolekül vier peptidfreie MHC-Klasse-I-Moleküle umfasst, die über nichtkovalente Wechselwirkungen zwischen Streptavidin und einem Biotin-Tag an jedem peptidfreien MHC-Klasse-I-Molekül an Streptavidin befestigt sind.

11. Zusammensetzung für ein Detektieren einer oder mehrerer auf antigenes Peptid ansprechender T-Zellen in einer Probe, die Zusammensetzung umfassend:
(a) mindestens zwei beladbare Detektionsmoleküle nach einem der Ansprüche 8-10, oder
(b) mindestens drei beladbare Detektionsmoleküle, die jeweils mindestens ein peptidfreies MHC-Klasse-I-Molekül und mindestens eine detektierbare Markierung umfassen, wobei jedes beladbare Detektionsmolekül eine unterschiedliche detektierbare Markierung umfasst,
wobei das peptidfreie MHC-Klasse-I-Molekül eine schwere Kette umfasst, die eine alpha-1-Domäne und eine alpha-2-Domäne umfasst, die durch eine Disulfid-Brücke verbunden sind, wobei die schwere Kette eine Aminosäuresequenz umfasst, die ausgewählt ist aus:
(a) SEQ ID-NR.: 12, oder
(b) einer Aminosäuresequenz mit mindestens 80 %, 85 %, 90 % oder 95 % Sequenzidentität zu der Sequenz in (a),
wobei ein mutierter Cysteinrest in der alpha-1-Domäne an Aminosäurerest 84 oder 85 positioniert ist und ein mutierter Cysteinrest in der alpha-2-Domäne an Aminosäurerest 139 positioniert ist.

12. Verwendung eines beladbaren Detektionsmoleküls nach einem der Ansprüche 8-10 oder einer Zusammensetzung nach Anspruch 11 für ein Detektieren einer oder mehrerer auf ein antigenes Peptid ansprechender T-Zellen in einer Probe.

13. Kit für ein Detektieren einer oder mehrerer auf antigenes Peptid ansprechender T-Zellen in einer Probe, das Kit umfassend:
i. ein beladbares Detektionsmolekül nach einem der Ansprüche 8-10 oder Zusammensetzung nach Anspruch 11,
ii. mindestens ein antigenes Peptid und
iii. optional Hinweise zur Verwendung.

14. Verfahren für Bestimmen der Wechselwirkung zwischen einem T-Zell-Rezeptor (TCR) oder einer auf antigenes Peptid ansprechenden T-Zelle und einer Bibliothek antigener Peptide, das Verfahren die folgenden Schritte umfassend:
i. Bereitstellen beladbarer Detektionsmoleküle nach einem der Ansprüche 8-10,
ii. Bereitstellen einer Bibliothek antigener Peptide,
iii. Inkontaktbringen der beladbaren Detektionsmoleküle mit der Bibliothek antigener Peptide, um eine Bibliothek beladener Detektionsmoleküle bereitzustellen, die Peptid-MHC(pMHC)-Klasse-I-Moleküle umfassen,
iv. Inkontaktbringen des T-Zell-Rezeptors (TCR) oder der auf antigenes Peptid ansprechenden T-Zelle mit der Bibliothek beladener Detektionsmoleküle und
v. Detektieren des Bindens des T-Zell-Rezeptors (TCR) oder der auf antigenes Peptid ansprechenden T-Zelle mit der Bibliothek beladener Detektionsmoleküle.

15. Verwendung eines beladbaren Detektionsmoleküls nach einem der Ansprüche 8-10 für ein Bestimmen der Wechselwirkung zwischen einem T-Zell-Rezeptor (TCR) oder einer auf antigenes Peptid ansprechenden T-Zelle und einer Bibliothek antigener Peptide.

## Revendications

1. Procédé de détection d'un ou plusieurs lymphocytes T sensibles à un peptide antigénique dans un échantillon, le procédé comprenant les étapes suivantes :
i. la fourniture de molécules de détection chargeables comprenant au moins une molécule de classe I du CMH (complexe majeur d'histocompatibilité) sans peptide et l'au moins une marque détectable,
ii. la fourniture de l'au moins un peptide antigénique,
iii. la mise en contact des molécules de détection chargeables avec l'au moins un peptide antigénique pour fournir des molécules de détection chargées comprenant l'au moins une molécule de classe I peptide-CMH (pCMH),
iv. la mise en contact des molécules de détection chargées avec l'échantillon, et
v. la détection de la liaison des molécules de détection chargées aux un ou plusieurs lymphocytes T sensibles à un peptide antigénique,
dans lequel chacun de l'au moins un peptide antigénique est représenté par :
- l'au moins deux marques détectables différentes, ou
- l'au moins une marque détectable qui est une marque d'acide nucléique,
dans lequel la molécule de classe I du CMH sans peptide comprend une chaîne lourde comprenant un domaine alpha-1 et un domaine alpha-2 liés par un pont disulfure, ladite chaîne lourde comprenant une séquence d'acides aminés choisie parmi :
(a) SEQ ID NO : 12, ou
(b) une séquence d'acides aminés présentant au moins 80 %, 85 %, 90 % ou 95 % d'identité de séquence avec la séquence en (a),
dans lequel un résidu de cystéine mutante est positionné dans le domaine alpha-1 au niveau du résidu d'acide aminé 84 ou 85 et un résidu de cystéine mutante est positionné dans le domaine alpha-2 au niveau du résidu d'acide aminé 139.

2. Procédé selon la revendication 1, dans lequel la molécule de classe I du CMH sans peptide est fixée à une molécule de jonction par des interactions non covalentes entre la molécule de jonction et une étiquette d'affinité sur la molécule de classe I du CMH sans peptide.

3. Procédé selon l'une quelconque des revendications précédentes, dans lequel la molécule de détection chargeable comprend les au moins deux molécules de classe I du CMH sans peptide, telles que les au moins trois molécules de classe I du CMH sans peptide, de préférence quatre molécules de classe I du CMH sans peptide.

4. Procédé selon l'une quelconque des revendications 2 et 3, dans lequel l'au moins une marque détectable est fixée à la molécule de jonction.

5. Procédé selon l'une quelconque des revendications précédentes, dans lequel l'au moins un peptide antigénique est représenté par les au moins deux marques détectables différentes, et la liaison des molécules de détection chargées aux un ou plusieurs lymphocytes T sensibles à un peptide antigénique est détectée en détectant la présence des au moins deux marques détectables différentes liées à un lymphocyte T sensible à l'antigène par l'intermédiaire des molécules de détection chargées.

6. Procédé selon la revendication 5, dans lequel les au moins deux marques détectables différentes sont choisies dans le groupe constitué par les marqueurs fluorescents, les radio-isotopes, les marqueurs magnétiques et les marques métalliques.

7. Procédé selon l'une quelconque des revendications 1 à 4, dans lequel l'au moins une marque détectable est une marque d'acide nucléique comprenant
i. une première région d'amorce en 5' (avant), une région de code à barres et une deuxième région d'amorce en 3' (arrière), et
ii. une région d'identifiant moléculaire unique (UMI) de bases nucléotidiques aléatoires
dans lequel la région de code-barres est un code à barres unique servant d'étiquette d'identification pour la molécule de détection.

8. Molécule de détection chargeable comprenant l'au moins une molécule de classe I du CMH sans peptide et une marque d'acide nucléique,
dans laquelle la molécule de classe I du CMH sans peptide comprend une chaîne lourde comprenant un domaine alpha-1 et un domaine alpha-2 liés par un pont disulfure, ladite chaîne lourde comprenant une séquence d'acides aminés choisie parmi :
(a) SEQ ID NO : 12, ou
(b) une séquence d'acides aminés présentant au moins 80 %, 85 %, 90 % ou 95 % d'identité de séquence avec la séquence en (a),
dans laquelle un résidu de cystéine mutante est positionné dans le domaine alpha-1 au niveau du résidu d'acide aminé 84 ou 85 et un résidu de cystéine mutante est positionné dans le domaine alpha-2 au niveau du résidu d'acide aminé 139.

9. Molécule de détection chargeable selon la revendication 8, dans laquelle la marque d'acide nucléique est telle que définie dans la revendication 7.

10. Molécule de détection chargeable selon l'une quelconque des revendications 8 ou 9, dans laquelle la molécule de détection chargeable comprend quatre molécules de classe I du CMH sans peptides fixées à la streptavidine par des interactions non covalentes entre la streptavidine et une étiquette de biotine sur chaque molécule de classe I du CMH sans peptides.

11. Composition pour la détection d'un ou plusieurs lymphocytes T sensibles à un peptide antigénique dans un échantillon, la composition comprenant :
(a) l'au moins deux molécules de détection chargeables selon l'une quelconque des revendications 8 à 10, ou
(b) l'au moins trois molécules de détection chargeables comprenant chacune l'au moins une molécule de classe I du CMH sans peptide et l'au moins une marque détectable, dans laquelle chaque molécule de détection chargeable comprend une marque détectable différente,
dans laquelle la molécule de classe I du CMH sans peptide comprend une chaîne lourde comprenant un domaine alpha-1 et un domaine alpha-2 liés par un pont disulfure, ladite chaîne lourde comprenant une séquence d'acides aminés choisie parmi :
(a) SEQ ID NO : 12, ou
(b) une séquence d'acides aminés présentant au moins 80 %, 85 %, 90 % ou 95 % d'identité de séquence avec la séquence en (a),
dans laquelle un résidu de cystéine mutante est positionné dans le domaine alpha-1 au niveau du résidu d'acide aminé 84 ou 85 et un résidu de cystéine mutante est positionné dans le domaine alpha-2 au niveau du résidu d'acide aminé 139.

12. Utilisation d'une molécule de détection chargeable selon l'une quelconque des revendications 8 à 10 ou d'une composition selon la revendication 11 pour la détection d'un ou plusieurs lymphocytes T sensibles à un peptide antigénique dans un échantillon.

13. Kit pour la détection d'un ou plusieurs lymphocytes T sensibles à un peptide antigénique dans un échantillon, le kit comprenant :
i. une molécule de détection chargeable selon l'une quelconque des revendications 8 à 10 ou une composition selon la revendication 11,
ii. au moins un peptide antigénique, et
iii. facultativement, des instructions d'utilisation.

14. Procédé pour déterminer l'interaction entre un récepteur de lymphocytes T (TCR) ou un lymphocyte T sensible à un peptide antigénique et une banque de peptides antigéniques, le procédé comprenant les étapes suivantes :
i. la fourniture de molécules de détection chargeables selon l'une quelconque des revendications 8 à 10,
ii. la fourniture d'une banque de peptides antigéniques,
iii. la mise en contact des molécules de détection chargeables avec la banque de peptides antigéniques pour fournir une banque de molécules de détection chargées comprenant des molécules de classe I peptide-CMH (pCMH),
iv. la mise en contact du récepteur de lymphocytes T (TCR) ou du lymphocyte T sensible à un peptide antigénique avec la banque de molécules de détection chargées, et
v. la détection de la liaison du récepteur de lymphocytes T (TCR) ou du lymphocyte T sensible à un peptide antigénique avec la banque de molécules de détection chargées.

15. Utilisation d'une molécule de détection chargeable selon l'une quelconque des revendications 8 à 10 pour déterminer l'interaction entre un récepteur de lymphocytes T (TCR) ou un lymphocyte T sensible à un peptide antigénique et une banque de peptides antigéniques.
